(19) Europäisches Patentamt
European Patent Office
Office européen des brevets

(11) Veröffentlichungsnummer: **0 330 725**
**A1**

(12) # EUROPÄISCHE PATENTANMELDUNG

(21) Anmeldenummer: **88110975.5**

(22) Anmeldetag: **08.07.88**

(51) Int. Cl.4: **C12N 15/00 , C12N 1/20 , C12P 21/02 , C07K 13/00 , A61K 37/02 , //(C12N1/20, C12R1:19)**

Die Bezeichnung der Erfindung wurde geändert (Richtlinien für die Prüfung im EPA, A-III, 7.3).

Der (Die) Mikroorganismus (Mikroorganismen) ist (sind) bei DSM unter der (den) Nummer(n) 4162,4163,4164,4165 hinterlegt worden.

(30) Priorität: **24.07.87 DE 3724581**

(43) Veröffentlichungstag der Anmeldung:
**06.09.89 Patentblatt 89/36**

(84) Benannte Vertragsstaaten:
**AT BE CH DE ES FR GB GR IT LI LU NL SE**

(71) Anmelder: **Grünenthal GmbH**
**Zieglerstrasse 6**
**D-5100 Aachen(DE)**

(72) Erfinder: **Gassen, Hans Günter, Prof., Dr., Dipl.-Chem.**
**Flachsbachweg 54**
**D-6100 Darmstadt(DE)**
Erfinder: **Machleidt, Werner, Prof., Dr.**
**Koboldstrasse 63**
**D-8000 München 83(DE)**
Erfinder: **Bartsch, Frank-Olaf, Dipl.-Biologe**
**Helfmannstrasse 60**
**D-6100 Darmstadt(DE)**
Erfinder: **Strauss, Michael, Dipl.-Ing.**
**Taunusstrasse 4**
**D-6140 Bensheim(DE)**

(74) Vertreter: **Patentanwälte Grünecker, Kinkeldey, Stockmair & Partner**
**Maximilianstrasse 58**
**D-8000 München 22(DE)**

(54) **Herstellung und Expression von DNA-Sequenzen, die für Proteine mit den biologischen Eigenschaften des Cystatin C kodieren.**

(57) Die Erfindung bezieht sich auf DNA-Sequenzen, die für Proteine mit den biologischen Eigenschaften des Cystatin C codieren, die Bereitstellung dieser DNA-Sequenzen, biotechnologische Verfahren zur Herstellung dieser Proteine und diese enthaltende pharmazeutische Zubereitungen.

DNA-Sequenzen, die für Proteine mit den biologischen Eigenschaften des Human-Cystatin C, einem Cystein-Proteinaseinhibitor aus 120 Aminosäuren, codieren und die Expression dieser Proteine mittels unterschiedlicher Vektoren in geeigneten Wirtsorganismen, insbesondere E.coli, werden beschrieben. Die Produkte werden, gegebenenfalls nach Abspaltung zusätzlicher Aminosäuren bzw. Proteine, als Fusionsproteine, natives Cystatin C oder auch als definierte Teilstücke erhalten. Die erhaltenen Proteine zeigen alle identische biologische Aktivität wie Cystatin C und eignen sich daher zum therapeutischen Einsatz bzw. zur Herstellung von Arzneimitteln, die diese Proteine als Wirkstoffe enthalten.

EP 0 330 725 A1

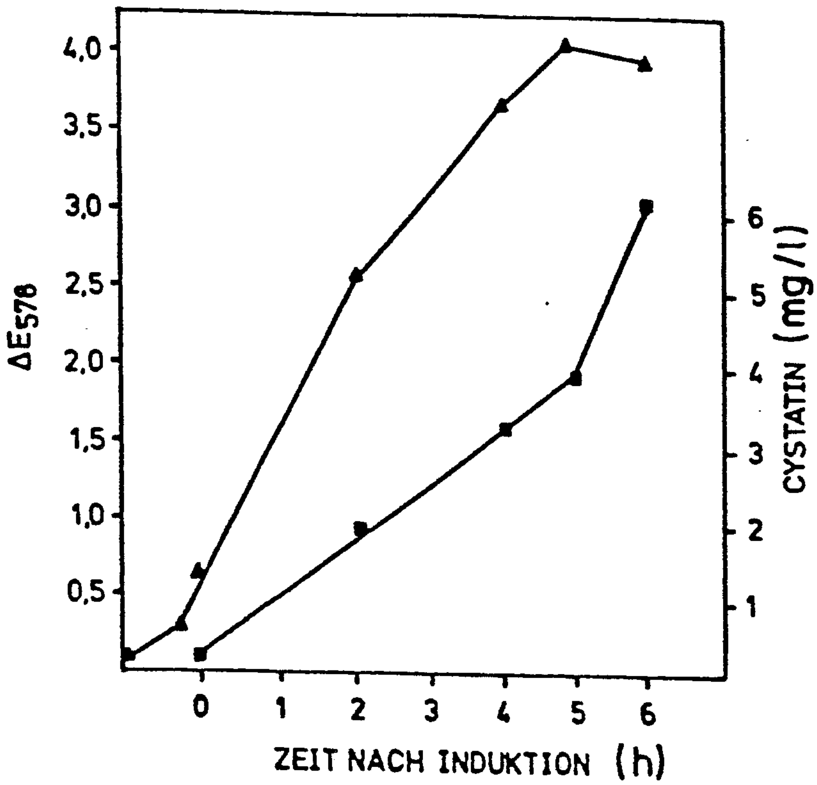


Fig. 8. Zeitlicher Verlauf von Zellwachstum und Cystatin-Expression nach Induktion mit ITPG in einer 8-l-Batchkultur.

Die Erfindung bezieht sich auf DNA-Sequenzen, die für Proteine mit den biologischen Eigenschaften des Cystatin C codieren, Bereitstellen der DNA-Sequenzen, rekombinante Vektoren zum Klonieren, die die genannten DNA-Sequenzen enthalten, Wirtsorganismen, die die genannten Vektoren enthalten sowie auf die exprimierten Proteine, biotechnologische Verfahren zum Herstellen dieser Proteine und diese enthaltende pharmazeutische Zubereitungen.

Zur Superfamilie der Cystein-Proteinaseinhibitoren gehören Proteine des Cystatin-Types, die aus etwa 95 bis 120 Aminosäuren bestehen. Aufgrund der Primärstrukturen wurden die Cystatine in Cystatin A, B und C gegliedert (Barret, 1984). Cystatin A, auch unter der Bezeichnung "Stefin" bekannt, findet sich vor allem in polymorphkernigen Leukozyten. Sein Hemmspektrum erstreckt sich auf die Cystein-Proteasen Papain, Kathepsin H und L sowie eingeschränkt auf Kathepsin B. Cystatin B ist in nahezu allen Zellen enthalten und läßt sich z.B. aus Lymphozyten und aus Monozyten isolieren. Cystatin C wurde sowohl im Blutserum als auch im menschlichen Seminalplasma und in Liquor cerebrospinalis nachgewiesen.

Das Human-Cystatin C konnte z.B. aus dem Blutserum von Patienten mit Autoimmunkrankheiten isoliert werden; es handelt sich um ein basisches Protein aus 120 Aminosäuren (Mg = 13 260) (Brzin, Popovic und Turk, 1984). Die Ermittlung der vollständigen Aminosäuresequenz von Cystatin C zeigte, daß diese mit der des seit längerer Zeit intensiv untersuchten Proteins "gamma-trace" (Grubb und Löfberg, 1982) identisch ist.

Cystatin C ist der wirksamste bisher bekannte Protein-Inhibitor für die Kathepsine B, H und L (Barret und Davies, 1984). Hinsichtlich der physiologischen Funktion von Cystatin C wird postuliert, daß das Protein bei der Regulation der Aktivität von lysosomalen Cystein-Proteinasen eine Rolle spielt.

Wie bereits einleitend gesagt, ist der Human-Cystein-Proteinaseinhibitor Cystatin C ein Protein aus 120 Aminosäureresten, dessen vollständige Aminosäuresequenz seit 1982 unter dem Namen gamma-trace bekannt ist (Grubb und Löfberg, 1982). Darin befindet sich an Position 106 die Aminosäure Serin. Abweichend hiervon wurde 1986 allerdings eine Aminosäuresequenz ermittelt, die an Position 106 die Aminosäure Tryptophan besitzt.

Als Cystein-Proteinaseinhibitoren eignen sich die Proteine beispielsweise zur Therapie bestimmter Virusinfektionen, z.B. benötigen Picornaviren zur Prozessierung ihrer Proteine zu reifen Hüllproteinen eine Cystein-Proteinase (Nicklin et al., 1986). In der europäischen Patentanmeldung 0 188 262 wird unter anderem die Verwendung von Hühnereiweiß-Cystatin zur Behandlung von Virusinfektionen beschrieben. Weitere therapeutische Anwendungsgebiete der erfindungsgemäß erhältlichen Proteine beziehen sich auf das Tumorgebiet. So ist bekannt, daß Cystein-Proteinasen bei der Metastasierung bestimmter Human-Karzinome eine große Rolle spielen (Graf, Baici und Sträuli, 1981; Mullins und Rohrlich, 1982; Mort, Leduc und Recklies, 1983) und daß Cystein-Proteinaseinhibitoren die Tumorinvasion inhibieren können (Turk et al., 1983). Des weiteren eignen sich die Cystein-Proteinaseinhibitoren zur Therapie von pathologisch gesteigerter extrazellulärer Proteolyse; so konnte z.B. die intradermale Arthusreaktion bei Kaninchen durch gleichzeitige Injektion des Cystein-Proteinaseinhibitors aus Hühnereiweiß signifikant vermindert werden (Keilova und Tomasek,1977).

Obwohl also therapeutische Anwendungsmöglichkeiten für Cystein-Proteinaseinhibitoren bekannt waren, konnten diese Substanzen, insbesondere Human Cystatin C , bislang in der Therapie nicht eingesetzt werden, da sie nicht in ausreichender Menge in hochreiner Form zur Verfügung standen.

Der Erfindung liegt die Aufgabe zugrunde, Proteine mit den biologischen Eigenschaften des Human-Cystatins C in ausreichenden Mengen und in hochreiner Form zur Verfügung zu stellen.

Die Aufgabe wird durch die Bereitstellung von DNA-Sequenzen, die für Proteine mit den biologischen Eigenschaften des Cystatin C codieren, gelöst.

Ausgehend von der bislang unveröffentlichten Aminosäuresequenz für Cystatin C, die an Position 106 die Aminosäure Tryptophan besitzt, kann über den genetischen Code eine synthetische DNA-Sequenz nach folgenden Gesichtspunkten abgeleitet werden:

1. Die Codonauswahl erfolgt anhand sog. "codon-usage"-Tabellen von stark exprimierten Proteinen in E.coli (Gouy und Gautier, 1982; Grosjean und Fiers, 1982), um die Gensequenz für eine Expression im Wirtssystem E.coli zu optimieren.

2. Für die Konzeption der synthetischen DNA-Sequenz wurden die im für die Klonierung vorgesehenen Vektor pUC 18 (DSM 3424) vorkommenden Restriktionsstellen wie folgt berücksichtigt. Unter Ausnützen der Variabilität des genetischen Codes wurden dabei solche Restriktionsschnittstellen in der Sequenz des Genes vermieden, die auch der Vektor pUC 18 (in der "multipurpose cloning site") enthält. So wurden zwei in der entsprechend der "codon usage" entworfenen Gensequenz vorkommende Hind III-Schnittstellen (AAGCTT) eliminiert, indem das Alanincodon "GCT" durch das ebenfalls für Alanin codierende Triplett "GCA" ersetzt wurde.

Die für Cystatin-C codierende synthetische DNA-Sequenz wurde außerdem um die für die Translationsinitiation und -termination notwendigen Sequenzen ergänzt. Dafür wurde vor das Codon für die erste, im reifen Protein gefundene Aminosäure das Startcodon ATG plaziert, im Anschluß an das für die letzte Aminosäure codierende Triplett wurden die beiden Stopcodons TAG und TAA eingefügt.

3. Zweckmäßigerweise wird die Sequenz des synthetischen Genes an den Enden durch Anfügen von Sequenzen, die den überstehenden Enden einer Restriktionsschnittstelle entsprechen, ergänzt, um eine gerichtete Klonierung des synthetischen DNA-Fragmentes zu ermöglichen.

Vorzugsweise wird N-terminal das überstehende Ende einer Bam HI-Schnittstelle (a) und C-Terminal das überstehende Ende einer Sal I-Schnittstelle (b) angefügt. Die Enden weisen dann folgende Sequenzen auf:

```
a) N-Terminus
5' GATCC ATG --- --- ---
3'     G TAC --- --- ---

b) C-Terminus
5' --- --- --- TAG TAA G
3' --- --- --- ATC ATT CAGCT
```

Die vor der für das reife Cystatin C codierenden DNA-Sequenz zusätzlich eingeführten Basen resultieren bei Expression eines Fusionsproteines in der Translation dreier zusätzlicher Aminosäuren ($Gly^{-3}$, $Ser^{-2}$ und $Met^{-1}$) vor dem N-Terminus des reifen Cystatin C.

Die Berücksichtigung von Shine-Dalgarno- und Promotor-Sequenzen im Gensynthesekonzept ist weniger zweckmäßig, da beim Fehlen derartiger Sequenzen die Möglichkeit besteht, das so konzipierte, herzustellende Cystatin-C-Gen als reines Strukturgen oder auch als Fusionsgen zu exprimieren. Abb.1 zeigt die der Genzynthese und der Klonierung gemäß der in den Beispielen erläuterten Vorgehensweise mit pUC 18 zugrundeliegende DNA-Sequenz. Die Länge des konzipierten Strukturgens beträgt 375 bp.

Abb.2 zeigt schematisch die Strategie der Gensynthese. Die Ausbildung des DNA-Doppelstranges über Wasserstoffbrücken-Bindungen der komplementären Basen erfolgt durch eine "annealing"-(Hybridisierungs)-Reaktion der 5′-phosphorylierten Oligonucleotide. Die Temperaturführung wird dabei so gewählt, daß das thermodynamisch stabilste Produkt, also das Gen mit der richtigen DNA-Sequenz, entsteht. Dazu wird der Reaktionsansatz auf eine Temperatur von etwa 95 °C bis 100 °C gebracht und über einen Zeitraum von etwa 16 Stunden auf etwa 15 °C bis 20 °C abgekühlt. Nach Zugabe des entsprechend den terminalen Enden des Gens mit Bam HI/Sal I hydrolysierten sowie dephosphorylierten Vektors pUC 18 erfolgt die Knüpfung der Phosphodiester-Bindungen mit dem Enzym T4-DNA-Ligase.

Mit dem hier dargelegten Schema der DNA-Synthese können DNA-Sequenzen hergestellt werden, die für Cystatin C, Fusionsproteine oder biologisch mit Cystatin C gleichwirkende Teilproteine des Cystatin C codieren.

Um das chemisch synthetisierte Cystatin C-Gen als Fusionsgen in E.coli zu exprimieren und Cystatin C in E.coli auf Proteinebene nachzuweisen, kann das Gen in Leserasterrichtung mit dem C-Terminus des Gens für β-Galactosidase in einem geeigneten Vektor verknüpft werden. Das Fusionsprotein läßt sich durch immunologische Reaktion mit Anti-Cystatin-C-Antiserum nachweisen.

Zur Synthese nativer Cystatine wird das Strukturgen mit der Signalsequenz des Phosphatase-A-Gens verbunden (Kikuchi et al., 1981). Bekanntlich bewirkt diese Signalsequenz von 21 Aminosäuren, daß das resultierende Präenzym in den periplasmatischen Raum der Bakterien ausgeschleust wird. Die Signalsequenz wird beim Durchgang durch die Plasmamembran mit Hilfe der Signalpeptidase abgespaltet. Das native Protein oder seine Prozessierungsprodukte erscheinen im Periplasma und partiell im Zellüberstand (Dodt et al., 1986).

Ein im E.coli zur Expression gebrachtes rekombinantes Human-Cystatin-C unterscheidet sich von der Primärstruktur des nativen Proteins dadurch, daß hier, bedingt durch die besondere Konstruktion des Expressionsvektors, am N-Terminus drei zusätzliche Aminosäuren eingebaut worden sind. Auch dieses Protein weist die biologischen Eigenschaften des Cystatin C auf.

Eine weitere spezielle Ausführungsform, die nach wie vor die Funktion des Human-Cystatin-C aufweist,

weist die Aminosäuresequenz

LEU VAL GLY GLY PRO MET ASP ALA SER VAL GLU GLU GLU GLY
VAL ARG ARG ALA LEU ASP PHE ALA VAL GLY GLU TYR ASN LYS
ALA SER ASN ASP MET TYR HIS SER ARG ALA LEU GLN VAL VAL
ARG ALA ARG LYS GLN ILE VAL ALA GLY VAL ASN TYR PHE LEU
ASP VAL GLU LEU GLY ARG THR THR CYS THR LYS THR GLN PRO
ASN LEU ASP ASN CYS PRO PHE HIS ASP GLN PRO HIS LEU LYS
ARG LYS ALA PHE CYS SER PHE GLN ILE TYR ALA VAL PRO TRP
GLN GLY THR MET THR LEU SER LYS SER THR CYS GLN ASP ALA

auf. Die funktionelle Vergleichbarkeit dieses sogenannten $Leu^{+9}$-Fragments läßt sich durch Abschätzung der Werte der Inhibitorkonstanten (KI) für Human-Cystatin-C sowie für das $Leu^{+9}$-Fragment nach der Methode von Green und Work (1952) zeigen.

Die DNA-Sequenz, die für das rekombinante, drei zusätzliche Aminosäuren enthaltende Cystatin-C codiert, ist in Abb.1 dargestellt.

Die bisher geschilderten DNA-Sequenzen können in geeig nete Vektoren inseriert werden.

Vorzugsweise wird zum Klonieren der Vektor pUC 18, der auch zur Sequenzierung geeignet ist, verwendet. pUC 18 enthält ein Ampicillin-Resistenzgen und eine "multi-purpose cloning site", die in der Sequenz für das alpha-Peptid der $\beta$-Galactosidase lokalisiert ist. Durch Insertion des Cystatin-C-Gens in die "multi-purpose cloning site" läßt sich das alpha-Peptid-Gen inaktivieren. Dazu wird aus pUC 18 durch Bam HI/Sal I-Hydrolyse ein 11-bp-Fragment entfernt (siehe Abb.2).

Bevorzugte Plasmide, die die zur Expression geeigneten DNA-Sequenzen, die für Proteine codieren, die die biologischen Eigenschaften des Cystatin C aufweisen, enthalten, sind die Plasmide pUR 288 C (DSM 4164), pMS 103 (DSM 4165), sowie pUC 18 C (DSM 4163), die in Übereinstimmung mit den Hinterlegungsmodalitäten des Budapester Vertrags bei der durch den Budapester Vertrag anerkannten Hinterlegungsstelle "Deutsche Sammlung für Mikroorganismen" hinterlegt wurden.

Als Wirtsorganismen, die sich für die Expression eines Proteins mit den biologischen Eigenschaften des Cystatin C eignen, können Bakterien, vorzugsweise der Arten E.coli oder B.subtilis, oder mikroskopisch kleine Pilze, vorzugsweise der Arten Aspergillus niger, Aspergillus oryzae oder Saccharomyces cerevisiae, verwendet werden.

Bevorzugt wurden zur Transformation Stämme von E.coli, beispielsweise K12 JM 101 (ATCC 33876), K12 DH1 (ATCC 33849), K12 W6 (DSM 3632), sowie besonders bevorzugt K12 JM 105 (DSM 4162) verwendet.

Bei der Transformation von E.coli K12 JM 105 (DSM 4162) ergibt sich der verfahrenstechnische Vorteil, daß dieser Stamm für eine inaktive $\beta$-Galactosidase mit einer Deletion ( M15) im Bereich des alpha-Peptides codiert.

Die Selektion transformierter Zellen mit rekombinanten Plasmiden kann auf LB-amp-Platten mittels des X-Gal/IPTG-Farbumschlagsystems (Miller, 1972) erfolgen, wobei der Farbumschlag auf der Komplementation der inaktiven $\beta$-Galactosidase des E.coli-Wirtsstammes durch ein vom Vektor codiertes intaktes alpha-Peptid beruht. Zellen ohne Vektor können auf Ampicillin-haltigem Medium nicht wachsen.

- Zellen mit nicht vollständig hydrolysiertem (nur Bam HI- oder nur Sal I-hydrolysiertem) und deshalb religiertem Vektor bilden aufgrund der alpha-Komplementation blaue Kolonien.
- Zellen mit rekombinantem Vektor bilden weiße Kolonien.

Der Genotyp von E.coli JM 105 kann wie folgt definiert werden:
thi-l, rpsL, endA, sbcB15, hsdR4, (lac-proAB), [F' traD36, proAB, $lacI^q$ lac Z $\Delta$ M15](Yanish-Perron, Vieira und Messing, 1985).

Die Erfindung betrifft nun DNA-Sequenzen, die für Proteine mit den biologischen Eigenschaften des Cystatin C codieren, und die Bereitstellung dieser DNA-Sequenzen. Diese DNA-Sequenzen können für Cystatin C in Form des nativen Proteins, in Form von Fusionsproteinen, aber z.B. auch für aktive Teilsequenzen codieren. Ferner betrifft die Erfindung ein Verfahren zum Herstellen der vorstehend genannten Proteine, bei dem man einen mit einem die genannten DNA-Sequenzen enthaltenden Vektor transformierten Wirtsorganismus in einem üblichen Nähr medium züchtet, gegebenenfalls die Expression des

Genproduktes induziert, das Expressionsprodukt aus der Kultur, d.h. aus den kultivierten Zellen oder aus dem Nährmedium isoliert und gegebenenfalls durch Affinitätschromatographie und/oder High Performance Liquid Chromatographie/ Fast Performance Liquid Chromatographie (HPLC/FPLC) reinigt.

Durch die vorliegende Erfindung wird somit erstmals die Bereitstellung ausreichender Mengen von Proteinen mit den biologischen Eigenschaften des Human-Cystatins C in hochreiner Form ermöglicht. Dementsprechend sind Arzneimittel mit einem wirksamen Gehalt an einem Protein mit der biologischen Aktivitiät des Cystatin C und üblichen Träger- und/oder Hilfsstoffen und/oder Verdünnungsmitteln ebenfalls Gegenstand der vorliegenden Erfindung. Zur Therapie kann das Protein mit der biologischen Aktivität des Cystatin C in Form steriler isotonischer Lösungen durch intramuskuläre, intravenöse oder subkutane Injektionen oder gegebenenfalls durch Infusion verabfolgt werden. Es kommen aber auch Sprayformen, Pulverzubereitungen oder Tinkturen zur intranasalen Anwendung in Betracht und Arzneimittel, die zur Behandlung von Erkrankungen der Atemwege durch direktes Aufbringen der Wirkstoffe auf die betroffenen Teile der Bronchien und Lunge besonders geeignet sind, liegen in Spray- oder Inhalationsformen vor.

Die Erfindung wird im folgenden anhand der Beispiele und Figuren näher erläutert. Es zeigen die

Fig.1 DNA-Sequenz, die der Synthese und der Klonierung des Cystatin-C-Gens zugrunde gelegt wurde. Die Enden der 16 Oligonucleotide (C1-C16) sind durch Trennstriche gekennzeichnet. Die überlappenden Bereiche umfassen jeweils 13 bp.

Fig.2 Schema zur Synthesestrategie des Cystatin-C-Gens. Links oben sind die Oligonucleotide C1 bis C16 gezeigt. Ihr "annealing"-(Hybridisierungs)-Produkt weist ein Bam HI- und ein Sal I-Ende auf. Aus dem Vektor pUC 18 wurde durch Bam HI/Sal I-Hydrolyse ein 11-bp-Fragment entfernt (rechts). Ligation von hydrolysiertem pUC 18 und "annealing"-Produkt lieferte den rekombinierten Vektor pUC 18C (Schwarzer Balken = Ampicillin-Resistenzgen; schraffierter Balken = Cystatin-C-Gen.)

Fig.3 Sequenzen der 16 Oligonucleotide, die bei der Synthese des Cystatin-C-Gens verwendet wurden.

Fig.4 Schema zur Sequenzierungsstrategie von pUC 18C. Der schwarze Balken symbolisiert das Cystatin-C-Gen, die von ihm rechts und links ausgehenden Linien Anteile der "multipurpose cloning site". (BP = Basenpaare)

Fig.5 Schema zur Konstruktion von pUR 288C. (Schwarzer Balken = Ampicillin-Resistenzgen; gepunkteter Balken = $\beta$-Galactosidase-Gen (pUR 288, pUR 288C) bzw. alpha-Peptid-Sequenz des $\beta$-Galactosidase-Gens (pUC 18C); schraffierter Balken = Cystatin-C-Gen. Die Ableserichtung ist durch Pfeile gekennzeichnet.

Fig.6 Schema zur Konstruktion von pMS 103. Einzelheiten siehe Beispiel 3a. (Dicker schwarzer Balken = Ampicillin-Resistenzgen; dünner schwarzer Balken = "multi-purpose cloning site"; gepunkteter Balken = Transkriptions-Terminator (rrnB$T_1T_2$); gekreuzter Balken = tac-Promotor ($P_{tac}$); weit schraffierter Balken = Cystatin-C-Gen; eng schraffierter Balken = Signalsequenz für die Alkalische Phosphatase; B, BG, E,H und S = Schnittstellen für Bam HI, Bgl II, Eco RI, Hind III und Sal I. Die Ableserichtung ist durch Pfeile gekennzeichnet.)

Fig.7 Ausschnitt aus der DNA-Sequenz des Ligationsproduktes aus Beispiel 3a. Der Ausschnitt beginnt am 5′-Ende mit der Shine-Dalgarno-Sequenz (S/D) (AGGA) aus pKK 223-3. Die Aminosäuren Valin (VAL) bis zum letzten Alanin (ALA) gehören zur Signalsequenz der Alkalischen Phosphatase. Die beiden nächsten Aminosäuren Glycin (GLY) und Serin (SER) werden zusätzlich durch die Bam HI-Stelle eingebracht. Ab Methionin (MET) beginnt die Cystatin-C-Sequenz.

Fig.8 Zeitlicher Verlauf von Zellwachstum und Cystatin-Expression nach Induktion mit ITPG in einer 8-l-Batchkultur. ( ▲ = Durch Messung der optischen Dichte bei 578 nm ermitteltes Zellwachstum; ■ = im Papain-Inhibitionstest ermittelte Cystatin-Konzentration.)

Fig.9 Vollständige Aminosäuresequenz von rekombinantem Human-Cystatin-C aus E.coli. Am N-Terminus sind drei zusätzliche Aminosäuren eingebaut. (S = durch automatisierten Edman-Abbau ermittelte Sequenz des N-Terminus; B1, B2 = Bromcyan-Fragmente; T = nach Abbau durch Trypsin sequenziertes Fragment).

Die im Folgenden benutzten Abkürzungen haben die Bedeutungen:

$A_{578}$ Absorption bei 578 nm
ATP Adenosin-5′-triphosphat
BSA Rinderserumalbumin
bp Basenpaare
DMSO Dimethylsulfoxid
DTT Dithiothreitol
EDTA Äthylendiamintetraessigsäure

IgG Immunglobulin G
IPTG Isopropyl-β-D-thiogalactosid
LB-Medium Dieses enthält pro Liter: 10g Casein enzymatisch gespalten (Sigma), 5g Hefeextrakt (DIFCO), 10g NaCl. Der pH-Wert wurde auf 7,3 eingestellt mit 10 M NaOH; für LB-amp-Medium werden pro Liter 50 mg Ampicillin zugesetzt. Zur Herstellung von Agarplatten werden pro Liter 15g Agar zusätzlich eingesetzt.
NL Normliter
PTH Phenylthiohydantoin
RT Raumtemperatur
SDS Natriumdodecylsulfat
Tris Trishydroxymethylaminomethan
U Einheit der Enzymaktivität
X-Gal 5-Brom-4-chlor-3-indolyl-β-D-galactopyranosid.

In den Ausführungsbeispielen wird unter Verwendung der genannten Materialien nach den nachstehend beschriebenen Verfahrenzsweisen vorgegangen:

## Enzyme

In Klammern sind jeweils die Bezugsquellen angegeben:

- Restriktionsendonucleasen (Boehringer, Mannheim);
Bethesda Research Laboratories "BRL" und Biolabs)
- E.coli-DNA-Polymerase I (Klenow-Fragment) (Boehringer, Mannheim)
- Lysozym (Boehringer, Mannheim)
- Alkalische Phosphatase aus Kälberdarm (Boehringer, Mannheim)
- Ribonuclease A (BRL)
- T4-DNA-Ligase (BRL)
- T4-Polynucleotid-Kinase (BRL)
- Papain (Sigma)

Restriktionsendonucleasen, T4-DNA-Ligase und alkalische Phosphatase wurden, wenn nicht anders angegeben, nach den Angaben der Hersteller benutzt.

## Weitere Substanzen (soweit nicht anderweitig im vorliegenden Text erwähnt)

- anti-Cystatin-C-Antiserum aus Kaninchen (J.Stephan-Institut, Ljubljana)
- Cystatin aus Humanserum (J.Stephan-Institut, Ljubljana)
- anti-Kaninchen-IgG-Peroxidase-Konjugat aus Ziege (Sigma)
- 5-Brom-4-chlor-3-indolyl-β-D-galactopyranosid (= X-Gal) (Biomol)

## Methoden

### Gelelektrophorese und Gelelution

Proteine wurden in SDS-Polyacrylamid-Gelen nach Laemmli (1970) getrennt.

DNA-Fragmente wurden mit Agarose- oder, in Abhängigkeit von der Fragmentgröße, mit Polyacrylamid-Gelen aufgetrennt (Maniatis, Fritsch und Sambrook, 1982). Agarose-Gele: TAE-Puffer (40mM Tris-Acetat, pH 8,3, 2 mM EDTA); Polyacrylamid-Gele (Acrylamid/Bisacrylamid 19:1): TBE-Puffer (60 mM Tris-Base, 60 mM Borsäure, 1 mM EDTA, pH 8,3).

- Oligonucleotide und DNA-Fragmente mit einer Größe unter 500 bp wurden in 5 - 15%igen Polyacrylamid-Gelen getrennt.
- DNA-Fragmente, deren Größe mehr als 500 bp betrug, wurden in 0,8%igen "Low Melting Point"-Agarose-Gelen (0,5 ug Ethidiumbromid/ml) getrennt. Die Elution der Fragmente erfolgte nach Maniatis, Fritsch und Sambrook (1982).
- In analytischem Maßstab phosphorylierte Oligonucleotide wurden in denaturierenden Gelen (7 M Harnstoff), in präparativem Maßstab phosphorylierte Oligonucleotide wurden in nativen Gelen getrennt. Die Elution der Oligonucleotide erfolgte nach Maxam und Gilbert (1980). Abweichend hiervon erfolgte die Isolierung von Oligonucleotiden zur Gensynthese aus den Gelstücken durch Elektroelution in einer Biotrap®-Apparatur der Firma Schleicher und Schüll nach Vorschrift des Herstellers.

Oligonucleotidsynthesen:

Sämtliche Oligonucleotide zur Gensynthese, der Standard-"forward primer" 5′TCCCAGTCACGACGT 3′, der Standard-"reverse primer" 5′AACAGCTATGACCATG 3′ und die Oligonucleotide SP5 und SP6 (vgl. Beispiel 3a) wurden nach dem Festphasen-Phosphitverfahren (Caruthers, 1982) mit einem DNA-Synthesizer (Typ 380 A) der Firma Applied Biosystems hergestellt. Die Reinigung der Oligonucleotide wurde standardisiert mittels "reversed phase"- HPLC mit Shandom-Hypersil/ODS (ODS = Oktadeka-Silan) (Partikelgröße 5 µm, Säulengröße 4,6 mm x 250 mm) nach McLaughlin und Krusche (1982) durchgeführt. Das DMTr-Oligonucleotidgemisch (DMTr = 4,4′-Dimethoxytrityl) wurde zur Trockne eingeengt und durch Behandlung mit 500 µl 80%iger Essigsäure (Kontaktzeit: 20 min) detrityliert. Nach Entfernen der Essigsäure im Vakuum wurde mit dem o.g. Säulenmaterial rechromatographiert. Die dermaßen gereinigten Oligonucleotide wurden nach Phosphorylierung an den 5′-Enden mittels Elektrophorese in 8 - 15%igen Polyacrylamid-Gelen analysiert.

5′-Phosphorylierung von Oligonucleotiden

Die chemisch synthetisierten Oligonucleotide wurden in Polynucleotid-Kinase-Puffer (50 mM Tris-HCl, pH 8,0, 10 mM $MgCl_2$, 10 mM DTT) an den 5′-Enden analytisch bzw. präparativ phosphoryliert. Die Reaktionsansätze hatten folgende Zusammensetzung:

| Substanz | Analytischer Ansatz | Präparativer Ansatz |
|---|---|---|
| Oligonucleotide | 5 pMol | 20 pMol |
| ATP | 20 pMol | 100 pMol |
| [$\gamma$-$^{32}$P] ATP (8.000 Ci/mMol) | 1 pMol | 5 pMol |
| T4-Polynucleotid-Kinase | 0,2 U | 5 U |

Die Reaktion erfolgte bei 37° C. Analytische Ansätze (Volumen: 10 µl) wurden 10 Minuten, präparative Ansätze (Volumen: 20 µl) 20 Minuten inkubiert. Die Trennung von Oligonucleotiden und ATP/[$\gamma$-$^{32}$P] ATP erfolgte durch Polyacrylamid-Gelelektrophorese. Die Banden wurden mittels Autoradiographie sichtbar gemacht. Die Gelstücke wurden ausgeschnitten und die Phosphorylierungsausbeuten durch Messung der Cerenkow-Strahlung im Tritium-Kanal eines Tricarb 3330-Szintillationszählers der Firma Packard nach Vorschrift des Herstellers bestimmt.

G-50-Gelchromatographie

Oligonucleotidgemische wurden durch G-50-Gelchromatographie entsalzt, und phosphorylierte Oligonucleotide, die nicht mittels Polyacrylamid-Gelelektrophorese gereinigt worden waren, wurden durch G-50-Gelchromatographie von ATP/[$\gamma$- $^{32}$P] ATP getrennt. Die Säule wurde frisch angesetzt unter Verwendung einer silikonisierten Pasteurpipette und Sephadex G-50 fine (Firma Pharmacia). Das Gelmaterial wurde nach Angaben des Herstellers behandelt. Die auf ein Volumen von 50 µl eingeengten Oligonucleotidlösungen wurden auf die Säule aufgetragen. Die Elution erfolgte mit Wasser und wurde mittels eines Geigerzählers kontrolliert. Die ersten 15 Tropfen, die Radioaktivität zeigten, wurden in einem Eppendorfgefäß vereinigt.

Präparation von Plasmid-DNA

Die Präparation aus 500-ml-Kulturen erfolgte nach Hardis et al. (1979) (pUC 18C und pUR 288) bzw. nach Hillen, Klein und Wells (1981) (pUC 18, pMS 103 und pKK 223-3). Plasmid-Schnellpräparationen

wurden nach Birnboim und Doly (1978) oder nach Willimzig (1985) durchgeführt.

## DNA-Sequenzanalysen

Die DNA-Sequenzierung beruhte auf der Methode von Sanger, Micklen und Coulson (1977) und wurde nach Korneluk, Quan und Gravel (1985) durchgeführt.

## Transformation von E.coli

Die Transformation von E.coli wurde mit $CaCl_2$-vorbehandelten Zellen nach Cohen und Chang (1972) durchgeführt. Bei der Transformation mit rekombinanten pUR 288 (hergestellt entsprechend Beispiel 2a) wurden DMSO-vorbehandelte Zellen nach Yanish-Perron, Vieira und Messing (1985) transformiert. Wenn nicht anders angegeben, wurden zur Transformation standardmäßig 10 -20 ng Plasmid-DNA (bezogen auf in den Ligase-Reaktionen eingesetzten Vektor) eingesetzt.

## Immunologische Identifizierung filtergebundener Proteine

Die Immundetektion von filtergebundenem Protein nach Durchführung einer Western-Blot-Analyse (Towbin, Staehlin und Gordon, 1979) erfolgte durch Inkubation mit einem verdünnten Kaninchen-anti-Cystatin-C-Antiserum und anschließende Bindung von Ziege-anti-Kaninchen-IgGs, die kovalent mit dem Enzym Peroxidase gekoppelt waren. Die Enzymreaktion von Peroxidase wurde mit dem Substrat 1,4-Chlornaphthol durchgeführt. Die einzelnen Inkubationsschritte sind im Folgenden stichpunktartig aufgelistet; die angegebenen Volumina beziehen sich auf einen 15 cm x 16 cm großen Filter.

1.Vorinkubation 1h, 37° C, in 100 ml "blocking"-Puffer
2.Inkubation 2 h, RT, in 50 ml Erst-Antiserum
3.Waschen 2 x 10 min, RT, in je 100 ml Waschpuffer
4.Waschen 2 x 20 min, RT, in je 100 ml "blocking"-Puffer
5.Inkubation 1 h, RT, in 50 ml Zweit-Antiserum
6.Waschen 1 x 10 min, RT, in 100 ml Waschpuffer
7.Waschen 2 x 10 min, RT, in 60 ml "blocking"-Puffer
8.Waschen 1 x 10 min, RT, in 100 ml Waschpuffer
9.Substratreaktion Inkubation für 20 min, RT, in 50 ml Substratlösung
10.Waschen 2 x 15 min, RT, in 100 ml Wasser
11. Filter trocknen und zwischen saugfähigem Filterpapier kühl und dunkel aufbewahren.

Die dabei benutzten Puffer-, Antisera- bzw. Substratlösungen haben folgende Zusammensetzung:

"Blocking"-Puffer:

50 mM Tris-HCl, pH 7,4
200 mM NaCl
0,05 % Tween-20
3 % Gelatine, gepulvert
Zur Lösung der Gelatine wird das Gemisch vor Gebrauch 1 h bei 37° C gehalten.

Waschpuffer:

50 mM Tris-HCl, pH 7,4
200 mM NaCl

Erst-Antiserum:

Cystatin-C-Antiserum von Kaninchen 1 : 300 verdünnt in "blocking"-Puffer

Zweit-Antiserum

Kaninchen-IgG-Peroxidase-Konjugat aus Ziege, 1 : 1000 verdünnt in "blocking"-Puffer

Substrat-Lösung:

30 mg 1,4-Chlornaphthol werden in 10 ml Methanol gelöst. Zu 50 ml Waschpuffer gibt man 30 µl 30%ige Wasserstoffperoxid-Lösung. Diese beiden Lösungen sind unmittelbar vor der Inkubation anzusetzen und auf die Filter aufzugeben.

Reinigung von Cystatin C mittels Affinitätschromatographie

Human-Cystatin C wurde affinitätschromatographisch nach Brzin, Popovic und Turk (1984) gereinigt.

Papain-Inhibitionstest

Die biochemische Aktivität von Cystatin C wurde mit Hilfe der Inhibition von Papain nachgewiesen (Barrett, 1981).

Transfer von Proteinen auf Nitrocellulose

In SDS-Gelen getrennte Proteine wurden nach Towbin, Staehlin und Cordon (1979) auf Nitrocellulosefilter transferiert.

Die folgenden Beispiele schildern die erfindgungsgemäße Herstellung der für Proteine mit den biologischen Eigenschaften des Cystatin C codierenden DNA-Sequenzen und diese enthaltender Vektoren sowie die Expression von Cystatin C als Fusionsprotein, als rekombinantes, drei zusätzliche Aminosäuren enthaltendes Protein bzw. als natives Protein sowie biologisch aktiver Teilsequenzen des Human-Cystatin C und die Isolierung und Reinigung dieser Proteine. Die Beispiele dienen lediglich zur Erläuterung der Erfindung und haben keinerlei limitierende Bedeutung.

Beispiel 1

Entsprechend den vorstehend geschilderten Strategien wurde wie folgt das Cystatin-C-Gen synthetisiert, in pUC 18 inseriert, mit dem erhaltenen Vektor ein E.coli-Stamm transformiert und der rekombinante Plasmidvektor pUC 18C sequenziert.

a) Synthese des Cystatin-C-Gens

Die Synthese des kompletten Cystatin-C-Gens erfolgte nach dem ursprünglich von Khorana entwickelten sog. Eintopfligations-Verfahren (Brown und Belagaje, 1979). Die Darstellung der Doppelstrang-DNA für das Cystatin-C-Gen nach diesem Konzept erforderte die Synthese von vollständig überlappenden, an ihrem 5′-terminalen Ende phosphorylierten Oligonucleotiden. Hierzu wurde das Gen in 16 Oligonucleotide (C1 - C16) aus 38 bis 55 Nucleotiden unterteilt (vgl. Abb.1). Die überlappenden Bereiche waren jeweils 13 bp lang. In Abb.3 sind die Nucleotidsequenzen der 16 Oligonucleotide aufgeführt.

Die Synthese und Reinigung der Oligonucleotide C1 - C16 erfolgte in der oben bei "Methoden" beschriebenen Weise.

Da ein Autoradiogramm der Oligonucleotide nach analytischer Phosphorylierung mit [$\gamma$- $^{32}P$] ATP und Elektrophorese in einem denaturierenden 8%igen Polyacrylamid-Gel zeigte, daß ein Großteil der Oligonucleotide nicht ausreichend rein war, wurden die Oligonucleotide präparativ phosphoryliert und über ein natives 8%iges Polyacrylamid-Gel getrennt. Hierzu wurden jeweils 20 pMol Oligonucleotid mit dem

fünffachen Überschuß an ATP phosphoryliert, wobei [$\gamma$-$^{32}$P] ATP im Verhältnis 1 : 20 (bezogen auf ATP) zugegeben wurde. Bei der gelelektrophoretischen Trennung wurden nicht-phosphorylierte Oligonucleotide und ATP abgetrennt. Das Autoradiogramm der präparativen Polyacrylamid-Gelelektrophorese zeigte, daß die Phosphorylierungsausbeuten sehr unterschiedlich ausfielen. Diejenigen DNA-Banden, die gen größten Molekulargewichten entsprachen, wurden mit einem Skalpell aus dem Gel ausgeschnitten. Die Phosphorylierungsausbeuten der entsprechenden Oligonucleotide lagen zwischen 3 % und 60 %.

Die Gelstücke wurden vereinigt, in eine Elektroelutionsapparatur überführt, und die Oligonucleotide wurden aus der polymeren Gelmatrix eluiert. Die Oligonucleotide wurden anschließend über eine G-50-Gelfiltrationssäule entsalzt. Das Oligonucleotid C15 wurde ohne Auftrennung über ein Polyacrylamid-Gel direkt über eine G-50-Gelfiltrationssäule gereinigt und mit den restlichen Oligonucleotiden vereinigt.

Die Hybridisierung der Oligonucleotide zur Darstellung der Doppelstrang-DNA für das Cystatin-C-Gen wurde nach Standardmethoden in einem Volumen von 40 µl in 1 x Ligasepuffer (50 mM Tris-HCl, pH 7,4, 10 mM $MgCl_2$, 10 mM DTT, 1 mg BSA/ml), durchgeführt. Dazu wurde das Reaktionsgefäß 10 Minuten in einem Wasserbad (100 °C) erhitzt und anschließend über Nacht (16 h) auf Raumtemperatur abgekühlt. Zur Ausbildung der Wassertoffbrückenbindungen zwischen der Doppelstrang-DNA für das Cystatin-C-Gen und dem Vektor wurde dieser gleichzeitig in die Reaktionslösung gegeben.

b) Insertion des Cystatin-C-Gens in pUC 18, Transformation und Restriktionsanalyse der rekombinanten DNA

Die Eintopfligation des in Beispiel 1a erhaltenen Hydridisierungs-Produktes sowie des Vektors mit dem Hybridisierungs-Produkt wurde wie folgt durchgeführt: 20 µl des Hybridisierungs-Ansatzes, 0,8 ug Bam HI/Sal I-hydrolysierter und anschließend dephosphorylierter Vektor pUC 18 und 2 U T4-DNA-Ligase wurden bei einer ATP-Konzentration von 1 mM in 1x Ligasepuffer 16 h bei 12 °C inkubiert. Das Gesamtvolumen des Eintopfligations-Ansatzes betrug 30 µl.

Mit 15 µl des Eintopfligations-Ansatzes wurden E.coli K12 JM 105-Zellen transformiert. Die Zellen wurden auf LB-amp-Platten aufgetragen. Die Selektion erfolgte wie oben beschrieben in der von Miller (1972) angegebenen Weise. Insgesamt wurden 70 blaue und 24 weiße Kolonien erhalten. Zur Restriktionsanalyse wurden mit den weißen Kolonien DNA-Minipräparationen durchgeführt. Die dabei erhaltenen DNAs wurden in einem 1%igen Agarose-Gel nach ihrer Größe getrennt. Als mögliche positive Klone wurden solche angesehen, deren DNA ein höheres Molekulargewicht aufwies als der Vektor pUC 18. Je 2 µg Plasmid-DNA dieser Klone wurden einer Bam HI/Sal I-Hydrolyse unterzogen und die Fragmente wurden gelelektrophoretisch getrennt. Die ungefähre Größe der ausgeschnittenen Genfragmente konnte dabei durch Vergleich mit einem Markerstandard (Spp 1, Eco RI-hydrolysiert) ermittelt werden. Insgesamt erwiesen sich von 24 untersuchten weißen Klonen 6 in der Restriktionsanalyse als positiv. Rekombinante pUC 18-DNA (pUC 18C) aus einem der positiven Klone, die das Cystatin-C-Gen in der "multi-purpose cloning site" enthielt, wurde einer DNA-Sequenzierung unterzogen.

c) Sequenzierung des rekombinanten Plasmidvektors pUC 18 C

Diese Sequenzierung mit der Plasmid-DNA von Klon 22 wurde nach der Didesoxy-Terminationsmethode (Sanger, Micklen und Coulson, 1977, und Korneluk, Quan und Gravel, 1985) durchgeführt. Hierzu wurden je 20 µg pUC 18C-DNA mit Eco RI bzw. Hind III hydrolysiert. Nach Phenolextraktion wurden die beiden Restriktionsansätze einer Sperminpräzipitation unterzogen. Es wurde direkt aus hydrolysierter pUC 18C-Plasmid-DNA sequenziert; pro Sequenzieransatz wurde 1 µg (= 0,5 pMol) pUC 18C-DNA eingesetzt. Zur Hybridisierung mit denaturierter, Eco RI-hydrolysierter pUC 18C-DNA wurden als "primer" die Cystatin-C-Oligonucleotide C10 und C14 sowie Standard "forward-primer", je 9 pMol, verwendet. Entsprechend wurden zur Hybridisierung mit denaturierter, Hind III-hydrolysierter pUC 18C-DNA als "primer" die Cystatin-C-Oligonucleotide C3, C5 und C7 sowie Standard-"reverse primer", je 9 pMol, verwendet. Abb.4 zeigt die Sequenzierungsstrategie und die jeweiligen ermittelten DNA-Sequenzen. Durch die Vielzahl der Sequenzierungsansätze konnte die DNA-Sequenz des Cystatin-C-Gens in pUC 18C eindeutig bestätigt werden.

Beispiel 2

Expression von Cystatin-C als β-Galactosidase-Fusionsprotein

a) Konstruktion eines Fusionsgens im Plasmid pUR 288

Um das chemisch synthetisierte Cystatin-C-Gen als Fusionsgen in E.coli zu exprimieren und Cystatin C in E.coli auf Proteinebene nachzuweisen, wurde das Gen in Leserasterrichtung mit dem C-Terminus des Gens für β-Galactosidase in dem Plasmid pUR 288 (Rüther and Müller-Hill, 1983) verknüpft. Das Schema der Konstruktion ist in Abb.5 dargestellt.

10 µg des Plamids pUC 18C wurden mit den Restriktionsendonucleasen Bam HI und Sal I (je 15 U) hydrolysiert (Inkubation über Nacht in "high"-Puffer -100 mM NaCl, 50 mM Tris-HCl, pH 7,5, 10 mM $MgCl_2$ und 1 mM DTT - nach Maniatis et al., 1982). Die Fragmente wurden präparativ in einem 5%igen Polyacrylamid-Gel getrennt, und das 375-bp-Cystatin-C-Fragment wurde aus dem Gel eluiert. Auf die gleiche Art wurden 10 µg des Vektors pUR 288 mit Bam HI und Sal I hydrolysiert, dephosphoryliert und mit Phenol und Ether extrahiert. Nach Fällung mit Ethanol aus 0,3 M Kaliumacetatlösung wurde mit dem 375-bp-Fragment wie folgt ligiert: 0,2 pMol Fragment, 2 pMol Vektor und 1 U T4-DNA-Ligase wurden bei einer ATP-Konzentration von 1 mM über Nacht bei 4° C inkubiert. Das Gesamtvolumen des Ansatzes betrug 20 µl. Mit der Hälfte des Ligationsansatzes (10 µl) wurden DMSO-behandelte E.coli K12 JM 105-Zellen transformiert. Die Selektion der Klone erfolgte auf LB-amp-Agarplatten (Selekton auf Ampicillin-Resistenz). Es wurden 213 Klone erhalten; 12 davon wurden durch Plasmid-Schnellanalysen mit anschließender Bam HI- und Sal I-Restriktion in einem 1%igen Agarose-Gel untersucht. 9 der 12 Klone erwiesen sich als positiv, d.h. ihr Plasmid (pUR 288C) enthielt ein 375-bp-Bam HI/Sal I-Fragment.

b) Nachweis der Expression des Fusionsproteins in E.coli

Zur Induktion der Fusionsprotein-Expression wurden 200 ml LB-amp-Medium mit E.coli K12 JM 105/pUR 288C (DSM 4164) aus einer Übernachtkultur eines positiven Klons angeimpft. Die Kultur wurde bei 37° C inkubiert und das Wachstum der Zellen bei 578 nm verfolgt. Bei Erreichen einer Zelldichte von 0,5 $A_{578}$-Einheiten/ml wurde die Kultur auf 500 uM IPTG eingestellt und bei 37° C weiterinkubiert. Der zeitliche Verlauf der Expression des Fusionsproteins wurde anhand einer SDS-Plyacrylamid-Gelelektrophorese verfolgt. Bemerkenswert am Expressionsverlauf ist, daß nach der Induktion zunächst ausschließlich das Cystatin-C/β-Galactosidase-Fusionsprotein gebildet wurde und mit fortschreitender Inkubationsdauer (nach ca. 1h) auch β-Galactosidase entsteht.

Um das Fusionsprotein durch eine immunologische Reaktion mit anti-Cystatin-C-Antiserum nchzuweisen, wurden SDS-gelelektrophoretisch getrennte Proteine des E.coli-Rohextraktes auf Nitrocellulose transferiert (Western-Blot-Analyse). Das Nitrocellulose-Filter wurde 1 h bei 37° C mit "blocking"-Puffer inkubiert, um unspezifische Bindeplätze auf dem Filter abzusättigen (Lee et al., 1982). Dann wurde wie unter "Methoden (Immunologische Identifizierung filtergebundener Proteine)" beschrieben vorgegangen. Der Nachweis beruht hierbei auf der Reduktion von Wasserstoffperoxid, dem Substrat der Peroxidase, unter gleichzeitiger Oxidation von 1,4-Chlornaphthol zu gefärbten Reaktionsprodukten.

Beispiel 3

Expression eines rekombinanten Cystatin C in E.coli und Gewinnung dieses Proteins

a) Konstruktion des Expressionsvektors pMS 103

Das Plasmid pMS 103 ist ein Derivat des Expressionsvektors pKK 223-3 (de Boer und Comstock 1983), das hinter den Translationssignalen (tac-Promotor, Shine-Dalgarno-Sequenz) das Gen für die Signalsequenz der Alkalischen Phosphatase (phoA), eine zusätzliche Bam HI-Schnittstelle, das Cystatin-C-Strukturgen sowie einen starken ribosomalen RNA-Transkriptions-Terminator (Brosius et al., 1981) enthält. Die Konstruktion ist in Abb.6 dargestellt. Sie erforderte die Ligation von drei DNA-Fragmenten:

Fragment 1: pKK 223-3 wurde mit Eco RI/Hind III hydrolysiert, dabei wurde die "multi-purpose cloning site" entfernt. Reinigung von Fragment 1 erfolgte durch LMP-Agarose-Gelelektrophorese.

Fragment 2: Aus pUC 18C wurde das Cystatin-C-Gen mittels Bam HI/Hind III-Hydrolyse ausgeschnitten und durch LMP-Agarose-Gelelektrophorese gereinigt.

Fragment 3: Dieses 68 bp große Fragment lieferte die phoA-Sequenz (Kikuchi et al., (1981)). Es wurde durch Hybridisierung von zwei chemisch synthetisierten Oligonucleotiden (SP5, SP6) erhalten. Diese haben folgende Nucleotidsequenzen:

SP5

5' AATTGTGAAACAAAGCACTATTGCACTGGCACTCTTACCGTTACTGTTTA

CCCCTGTGACAAAAGCAG 3'

SP6

3' CACTTTGTTTCGTGATAACGTGACCGTGAGAATGGCAATGACAAATGGGG

ACACTGTTTTCGTCCTAG 5'

SP5 und SP6 wurden an ihren 5'-Enden nicht phosphoryliert, da sowohl Fragment 1 als auch Fragment 2 an den 5'-Enden phosphoryliert sind. Die Sequenzen von SP5 und SP6 wurden so gewählt, daß nach der Ligation mit dem Vektor die Eco RI-Stelle zerstört wird.

Ligation

Die Ligation der drei Fragmente wurde wie folgt durchgeführt. 20 µg Fragment 1 und ein je 10-facher molarer Überschuß an Fragment 2 und 3 wurden in 1 x Ligasepuffer unter Zusatz von ATP entsprechend einer Konzentration von 1 mM mit 1 U T4-DNA-Ligase für 16 h bei 4 °C inkubiert. Das Volumen des Ligationssatzes betrug 20 ul.

Abb.7 zeigt die DNA-Sequenz des Ligationsproduktes von der Shine-Dalgarno-Sequenz des tac-Promotors bis zum Start des Cystatin-C-Strukturgens.

b) Transformation und Restriktionsanalyse der rekombinanten DNA

Transformation

Nach Transformation von E.coli K12 JM 105 (nach Cohen und Chang, 1972) mit dem gesamten pMS 103-Ligationsansatz aus Beispiel 3a wurden die Zellen auf LB-amp-Platten aufgetragen. Die Selektion von Vektor-haltigen Zellen erfolgte über das Ampicillin-Resistenzgen von pKK 223-3. Zwischen Zellen mit Vektor und Zellen mit rekombinantem Vektor konnte in diesem System nicht unterschieden werden.

Restriktionsanalyse

Insgesamt wurden 160 weiße Klone erhalten. Aus 12 Klonen wurden DNA-Minipräparationen hergestellt. Eine Restriktionsanalyse (Eco RI-Hydrolyse, Bgl II-Hydrolyse) ergab folgendes:
2 Klone (Nr.4 und 6) enthielten pMS 103;
4 Klone (Nr.3,8,11,12) enthielten den re-ligierten Vektor pKK 223-3;
6 Klone (Nr.1,2,5,7,9,10) waren Deletionsmutanten mit einer Länge von ca. 2.700 bp (durch Vergleich mit Spp1-Marker abgeschätzt).

c) Papain-Inhibitionstest; Erzeugung von rekombinantem Cystatin C

Die biochemische Aktivität von Cystatin C wurde mit Hilfe der Inhibition von Papain nachgeweisen (Barrett, 1981).

Übernachtkulturen von E.coli K12 JM 105/pKK 223-3 sowie E.coli K12 JM 105 /pMS 103 (DSM 4165) (je 10 ml) wurden zum Beimpfen von 250 ml-Kulturen (LB-amp-Medium) verwendet. die Kulturen wurden

bei 37° C inkubiert, und das Wachstum der Zellen wurde bei 578 nm verfolgt. Bei Erreichen einer optischen Dichte von 0,5 - 0,7 $A_{578}$-Einheiten/ml wurden die Kulturen auf 500 µM IPTG eingestellt und bei 37° C unter Schütteln weiter inkubiert. Der zeitliche Verlauf der Induktion von Cystatin C wurde mittels des Papain-Inhibitionstestes verfolgt. Dabei zeigte sich, daß nur Kulturen, welche über das Plasmid pMS 103 verfügen, nach Induktion mit IPTG zur Produktion von Cystatin C befähigt sind. (Kontrollkulturen, welche an Stelle von pMS 103 das Ursprungsplasmid pKK 233-3 besaßen, zeigten im Inhibitionstest keine biochemische Aktivität.) Die im 250 ml-Maßstab erreichten Ausbeuten an Cystatin C liegen bei 4 mg/l; diese Proteinmengen wurden 5 - 6 h nach Induktion erreicht.

Zur Erzeugung größerer Quantitäten des rekombinanten Cystatin C wurden 8 l Batchkulturmedium (LB-amp-Medium) im Rührkesselfermenter Biostat E (Braun, Melsungen; Belüftungsrate 9 NL/min) mit 150 ml einer Übernachtkultur von E.coli K12 JM 105/pMS 103 beimpft. Die Kultur wurde bei 37° C inkubiert; das Zellwachstum wurde durch Messung der optischen Dichte ($A_{578}$-E/ml) verfolgt. Bei Erreichen von 0,6 $A_{578}$-E/ml wurde die Fermenterkultur auf 500 µM IPTG eingestellt und weiter inkubiert. Der zeitliche Verlauf von Zellwachstum und Cystatin-Produktion wurde verfolgt, wobei die Bestimmung der Cystatin-C-Mengen nach Barrett (1981) erfolgte. Die Zellen wurden hierzu durch Zentrifugieren über 3 Minuten abgetrennt und dann in 50 mM Tris-HCl/2 mM EDTA-Puffer, pH 8,0, mit Lysozym (10 mg/ml) aufgeschlossen (modifiziert nach Holmes und Quigley, 1981). Abb.8 zeigt, daß die Cystatin-C-Konzentration 6 h nach Induktion den Höchstwert von 6,2 mg/l erreicht. Dies entspricht einer Gesamtausbeute von 50,7 mg Cystatin aus 35,5 g Bakterien (Naßgewicht); die Zellen wurden 6 h nach Induktion durch Zentrifugation sedimentiert.

Um eine immunologische Reaktion (vgl.Beispiel 2b) mit anti-Cystatin-Antikörpern nachzuweisen, wurden SDS-gelelektrophoretisch getrennte Proteine sowohl des E.coli-Rohextraktes als auch des affinitätschromatographisch gereinigten Proteins (vgl. unten bei Beispiel 3d) auf Nitrocellulose transferiert. In beiden Fällen fand eine deutliche Immunreaktion des Antikörpers mit dem produzierten Protein statt.

d) Isolierung eines rekombinanten Human-Cystatin C aus E.coli

Zellaufschlußverfahren

Für einen periplasmatischen Zellaufschluß wurde 1 g abzentrifugierte Zellen in 50 ml Aufschlußmedium (20% Saccharose, 30 mM Tris-HCl, 1 mM EDTA, pH 8,0) mit 50 mg Lysozym bei Raumtemperatur für 60 min inkubiert (modifiziert nach Chan et al., 1981). Nach anschließender Zentrifugation enthielt der Überstand 1 mg des rekombinanten Cystatin C/50 ml Aufschlußmedium.

e) Reinigung des rekombinanten Cystatin C durch Affinitätschromatographie

Die Cysteinprotease Papain wurde nach Axen (1967) an mit Bromcyan aktivierte Sepharose 4B gebunden; anschließend erfolgte eine Carboxymethylierung der SH-Gruppen des Papains. Zur Reinigung des rekombinanten Cystatin C wurde der oben erhaltene Überstand des Zellaufschlusses bei 4° C für 1 h mit Carboxymethyl-Papain-Sepharose inkubiert. Nach Waschen des Säulenmaterials mit 10 mM Tris-HCl/1 M NaCl, pH 8,0, wurde das gewünschte Protein mit 10 mM NaOH eluiert (Brzin, Popovic und Turk, 1984). Die Chromatographie wurde mit Hilfe des Papain-Inhibitionstestes verfolgt. Cystatin-C-haltige Eluate wurden anschließend mit 2N HCl neutralisiert.

Aus 1 g Bakterien (Naßgewicht) konnten nach Affinitätschromatographie 0,6 mg des rekombinanten Human-Cystatin C erhalten werden.

f) HPLC-Trennung der affinitätschromatographisch gereinigten Fraktion

Das neutralisierte Affinitätssäuleneluat wurde ultrafiltriert (untere Ausschlußgröße des verwendeten Amicon YM10-Filters: 8000 Dalton). Das Retentat wurde durch Gelfiltration mit einer Sephadex G-25M-Säule (1,5 cm x 5 cm) entsalzt und anschließend lyophilisiert. Die so gewonnenen Proteine wurden in ca. 100 µl 0,1% Trifluoressigsäure (TFA) aufgenommen und mittels eines Acetonitril-Gradienten (40-60%) getrennt. (Chromatographie-Bedingungen: Partisil 5-$C_8$ = Säule (0,46 cm x 25 cm); Puffer A: 0,1% TFA in Wasser (v/v); Puffer B: 0,05% TFA in Acetonitril (v/v); Durchflußrate: 0,7 ml/min; Temperatur: 25° C). Zur HPLC-Trennung wurde ein Beckman-114 M solvent delivery-Modul mit einem 421 A-control system

verwendet; die Detektion erfolgte bei 280 nm mit einem Beckman 163 wavelength detektor. Die Proteinfraktionen 2, 3 und 4 zeigten im Papain-Inhibitionstest vergleichbare biochemische Aktivitäten. Diese Proteinfraktionen wurden wie folgt sequenziert:

g) Sequenzierung des aus E.coli isolierten rekombinanten Cystatin C

Zur Sequenzierung wurde ein Gasphasen-Sequenator (Applied Biosystems 470 A), der nach dem Prinzip des Edman-Abbaus arbeitet, verwendet. Die Analyse der PTH-Aminosäurederivate erfolgte ON-LINE über HPLC (Applied Biosystems 120 A). Um die N-terminalen Aminosäuren zu bestimmen, wurden die Proteine aus den HPLC-Fraktionen 2, 3 und 4 aus Beispiel 3f ansequenziert.

Die Analyse ergab, daß die Fraktionen unterschiedlich prozessierte Inhibitorproteine enthielten, die in Tabelle I zusammengestellt sind:

Tabelle I

| | N-terminale Aminosäure | Verhältnis Sequenz/Gesamtprotein |
|---|---|---|
| Fraktion 2 | $Gly^{-3}$<br>$Ser^{+1}$ | 20 %<br>10 % |
| Fraktion 3 | $Ser^{+1}$<br>$Gly^{+12}$ | 11 %<br>15 % |
| Fraktion 4 | $Leu^{+9}$ | 44 % |

Die Tabelle gibt die N-terminalen Aminosäuren der unterschiedlich prozessierten Inhibitorproteine und ihre relative Häufigkeit an. Die bei der Affinitätschromatographie erhaltene Proteinmenge wurde gleich 100% (= "Gesamtprotein") gesetzt. Aus dem Elutionsprofil der HPLC-Trennung wurden die %-Anteile der Proteine von Fraktion 2, 3 und 4, aus der Sequenzanalyse die %-Anteile der jeweiligen Proteinspezies (= "Sequenz") jeder Fraktion ermittelt.

Eines der Proteine, ein Protein aus Fraktion 2, das 20 % der Proteinmenge des Affinitätschromatographie-Eluats entspricht, wies die N-terminale Aminosäure auf, die aufgrund der durchgeführten Konstruktion zu erwarten war. Es handelt sich um das Protein, das am N-Terminus die Aminosäure $Gly^{-3}$ trägt (siehe dazu auch Abb.9). Durch die zusätzlich eingeführte Bam HI-Schnittstelle (vgl.Seite 4, Ziffer 3) werden zwischen der PhoA-Signalsequenz und dem Cystatin-Strukturgen die Aminosäuren $Gly^{-3}$, $Ser^{-2}$ und $Met^{-1}$ zur Expression gebracht. In an sich bekannter Weise (z.B. durch limitierte Bromcyan-Spaltung oder durch limitierte Spaltung mit Exopeptidase) können diese drei zusätzlichen N-terminalen Aminosäuren unter Gewinnung von Human-Cystatin C abgespalten werden.

Aufgrund der bereits bekannten Primärstruktur von Cystatin C konnte auf eine Sequenzierung nach dem Prinzip überlappender Fragmente weitgehend verzichtet werden.

Das Cystatin-Molekül besitzt nach Grubb und Löfberg (1982) zwei Disulfidbrücken. Zur weiteren Sequenzaufklärung wurde daher eine Bromcyan-Spaltung des Proteins durchgeführt (Gross, 1967). Die Bromcyan-Fragmente wurden durch HPLC (vgl. Beispiel 3f) getrennt und anschließend mit Hilfe des Gasphasen-Sequenators sequenziert. Da das Bromcyanfragment B2 über eine Disulfidbrücke zwischen $Cys^{97}$ und $Cys^{117}$ mit B1 verbunden ist, konnten beide Fragmente parallel sequenziert werden. Die Aminosäuresequenz von B2 ergibt sich aus den Sequenzierungsdaten nach der Subtraktion von B1.

Fehlende Restsequenzen wurden nach tryptischer Spaltung (Smyth, 1967) und HPLC-Trennung der Peptide sequenziert.

Abb.9 zeigt die ermittelte vollständige Aminosäuresequenz (Primärstruktur) des in E.coli zur Expression gebrachten rekombinanten Human-Cystatin C, die sich von der Primärstruktur des nativen Proteins nur dadurch unterscheidet, daß hier, bedingt durch die Konstruktion des Expressionsvektors, am N-Terminus die drei ersten Aminosäuren ($Gly^{-3}$, $Ser^{-2}$ und $Met^{-1}$) zusätzlich eingebaut worden sind. Durch Änderung der Konstruktion des Expressionsvektors (Auslassen der für die Konstruktion der Bam HI-Schnittstelle vor dem Translationsinitiationscodon notwendigen Nukleotide) läßt sich in der vorstehend beschriebenen Weise auch ein solches Gen erhalten und nach Einbau in entsprechende Vektoren in z.B. E.coli K12 JM 105 zur Expression bringen, das ausschließlich zu Human-Cystatin C der vollen Länge mit 120 Aminosäuren führt.

h) Durch Abschätzung der Werte der Inhibitorkonstanten ($K_i$) für Human-Cystatin C sowie für das

Leu$^{+9}$-Fragment (HPLC-Fraktion 4; vgl. Beispiel 3f und g) nach der Methode von Green und Work (1952) - die Bestimmung der freien Enzym-Konzentration erfolgte nach Barrett (1981) - konnte gezeigt werden, daß die apparente Inhibitorkonstante für beide Proteine kleiner als $10^{-9}$ M beträgt, die $K_I$-Werte für Human-Cystatin C und das Leu$^{+9}$-Fragment also vergleichbar sind.

Dieses Leu$^{+9}$-Fragment hat die Aminosäuresequenz:

LEU VAL GLY GLY PRO MET ASP ALA SER VAL GLU GLU GLU GLY
VAL ARG ARG ALA LEU ASP PHE ALA VAL GLY GLU TYR ASN LYS
ALA SER ASN ASP MET TYR HIS SER ARG ALA LEU GLN VAL VAL
ARG ALA ARG LYS GLN ILE VAL ALA GLY VAL ASN TYR PHE LEU
ASP VAL GLU LEU GLY ARG THR THR CYS THR LYS THR GLN PRO
ASN LEU ASP ASN CYS PRO PHE HIS ASP GLN PRO HIS LEU LYS
ARG LYS ALA PHE CYS SER PHE GLN ILE TYR ALA VAL PRO TRP
GLN GLY THR MET THR LEU SER LYS SER THR CYS GLN ASP ALA.

In der folgenden Tabelle II sind die hinterlegten Mikroorganismen zusammengefaßt:

Tabelle II

| Mikroorganismus | Hinterlegungsstelle | Hinterlegungsnr. |
|---|---|---|
| pUC 18 | DSM | 3424 |
| pBR 322 | ATCC | 31344 |
| pWH 701 | DSM | 3633 |
| E.coli K12 JM 101 | ATCC | 33876 |
| E.coli K12 DH1 | ATCC | 33849 |
| E.coli K12 W6 | DSM | 3632 |
| E.coli K12 JM 105 | DSM | 4162 |
| E.coli K12 JM 105/pMS 103 | DSM | 4165 |
| E.coli K12 JM 105/pUC 18C | DSM | 4163 |
| E.coli K12 JM 105/pUR 288C | DSM | 4164 |

## Literatur

Axen, R. (1967), Nature 214, 1302 - 1304.
Barrett, A.J. (1981), Meth.Enzymol. 80, 771 - 778.
Barrett, A.J. (1984), Intracellular Protein Catabolism V. Proceedings of the international symposium in Airlie, Virginia.
Barrett, A.J. und Davies, M. (1984), Biochem.Biophys.Res. Commun. 120, 631 - 636.
Brosius, J., Dull, T.J., Sleeter, D.D., und Noller, H.F. (1981) J. Mol. Biol. 148, 107 - 127.
Brown, E.L. und Belagaje, R. (1979), Meth. Enzymol. 68, 109 - 151.
Birnboim, H.C. und Doly, J. (1978), Nucl. Acids Res. 7, 1513 = 1523.
Brzin, J., Popovic, T. und Turk, V. (1984), Biochem. Biophys. Res.Commun. 118, 103 - 109.
Caruthers, M. H. (1982), Chemical and Enzymatic Synthesis of Gene Fragments (Gassen, H.G.und Lang, A., eds.) Seiten 97 - 102. Verlag Chemie, Weinheim -Deerfield Beach - Basel.
Chan, S., Weiss, J., Konrad, M., White, T., Ball, C., Yu, S.-D., Marks, D. und Steiner, D.F. (1981), Proc. Natl. Acad. Sci. 78, 5401 - 5405.
Cohen, S.N. und Chang, A.G.Y. (1972), Proc. Natl. Acad. Sci. 69, 2110 - 2114.
deBoer, H.A. und Comstock, L.J. (1983), Proc. Natl. Acad. Sci. 80, 21 - 25.
Dodt, J., Schmitz, T., Schäfer, T. und Bergmann, C., (1986), FEBS Letters 202, 373-377.
Gouy, M. und Gautier, C. (1982), Nucl.Acids Res. 10, 7055-7074.

Graf, M., Baici, A.B. und Sträuli, P. (1981) Lab.Invest. 45, 587-596.
Green, N.M. und Work, E. (1952), Biochem. J. 54, 347-352.
Grosjean, H. und Fiers, W. (1982), Gene 18, 199-209.
Gross, E. (1967), Meth. Enzymol. 11, 238-255.
Grubb, A. und Löfberg, H. (1982), Proc. Natl. Acad.Sci. 79, 3024-2027.
Hardies, S.C., Patient, R.K., Klein, R.D., Ho, F., Rezinkoff, W.S. und Wells, R.D. (1979), J. Biol. Chem. 254, 5527-5534.
Hillen, W., Klein, R.D. und Wells, R.D. (1981), Biochemistry 20, 3748-3756.
Holmes, D. und Quigley, M. (1981), Anal. Biochem.114, 193-197.
Keilova, H. und Tomasek, V. (1977), Acta biol. med. germ.36, 1873-1881.
Kikuchi, Y., Yoda, K., YamasaKl, M. UND Tamura, G., (1981), Nucleic Acid Res., 9, 5671-5678.
Korneluk, R.G., Quan, F. und Gravel, R.A. (1985), Gene 40, 317-323.
Laemmli, U.K. (1970), Nature 227, 680-685.
Lee, C.G., Huang, Y.S., Hu, P.C., Gomel, V. und Menge, A.C. (1982), Anal.Biochem. 123, 14-22.
Maniatis, T., Fritsch, E.F. und Sambrook, J. (1982): Molecular Cloning -A Laboratory Manual, Seite 104, Cold Spring Harbor Laboratory, Cold Spring Harbor, New York.
Maxam, A.M. und Gilbert, W. (1980), Meth.Enzymol. 65, 499-580.
McLaughlin, L.W. und Krusche, J.U. (1982), Chemical and Enzymatic Synthesis of Gene Fragments (Gassen, H.G. und Lang, A., eds.), Seiten 177-198. Verlag Chemie, Weinheim - Deerfield Beach - Basel.
Miller, J.H. (1972), Experiments in Molecular Gentics. Cold Spring Harbor Laboratory, Cold Spring Harbor, New York.
Mort, J.S., Leduc, M.S . und Recklies, A.D. (1983), Biochim.Biophys.Acta 755, 369-375.
Mullins, D.E. und Rohrlich, S.T. (1982), Biochem.Biophys.Acta 695, 177-214.
Nicklin, M.J.H., Toyoda, H., Murray, M.G. und Wimmer, E. (1986), Biotechnology 4, 33-42.
Rüther, U. und Müller-Hill, B. (1983), EMBO-J. 2, 1791-1794.
Sanger, F., Micklen,S. und Coulson, A.R. (1977), Proc.Natl.Acad.Sci. 74, 5463-5467.
Smyth, D. (1967), Meth. Enzymol. 11, 214-222.
Towbin, H., Staehlin, T. und Gordon, J. (1979), Proc. Natl. Acad. Sci. 76, 4350-4354.
Turk, V., Brizn, J., Kopitar, M., Kregar, I., Locnikar, P., Longer, M., Popovic, T., Ritonja, A., Vitale, Lj., Machleidt, W., Giraldi, T. und Sava, G. (1983), Proteinase Inhibitors: Medical and Biological Aspects (Katunuma, N. et al., eds), Seiten 125-134. Japan Scim Soc. Press, Tokyo, Springer Verlag, Berlin.
Willimzig, M. (1985), Trends in Genet. 1, 158.
Yanish-Perron, C., Vieira, J. und Messing J. (1985), Gene 33, 103-119.

## Ansprüche

1. DNA-Sequenzen, dadurch **gekennzeichnet,** daß sie für Proteine mit den biologischen Eigenschaften des Cystatin C codieren.

2. DNA-Sequenzen nach Anspruch 1, dadurch **gekennzeichnet,** daß sie synthetisch hergestellt werden.

3. DNA-Sequenzen nach einem der Ansprüche 1 oder 2, dadurch **gekennzeichnet,** daß sie für Cystatin-C-Fusionsproteine oder biologisch mit Cystatin C gleichwirkende Teilproteine des Cystatin C codieren.

4. DNA-Sequenzen nach einem der Ansprüche 1 bis 3, dadurch **gekennzeichnet,** daß sie für das Protein mit den biologischen Eigenschaften des Cystatin C mit der Aminosäuresequenz

LEU VAL GLY GLY PRO MET ASP ALA SER VAL GLU GLU GLU GLY
VAL ARG ARG ALA LEU ASP PHE ALA VAL GLY GLU TYR ASN LYS
ALA SER ASN ASP MET TYR HIS SER ARG ALA LEU GLN VAL VAL
ARG ALA ARG LYS GLN ILE VAL ALA GLY VAL ASN TYR PHE LEU
ASP VAL GLU LEU GLY ARG THR THR CYS THR LYS THR GLN PRO
ASN LEU ASP ASN CYS PRO PHE HIS ASP GLN PRO HIS LEU LYS
ARG LYS ALA PHE CYS SER PHE GLN ILE TYR ALA VAL PRO TRP
GLN GLY THR MET THR LEU SER LYS SER THR CYS GLN ASP ALA

codieren.

5. DNA-Sequenzen nach einem der Ansprüche 1 oder 2, dadurch **gekennzeichnet,** daß sie für das rekombinante, drei zusätzliche Aminosäuren enthaltende Cystatin C mit der in Abbildung 9 dargestellten Aminosäuresequenz codieren.

6. DNA-Sequenzen nach Anspruch 5, dadurch **gekennzeichnet,** daß sie die in Abbildung 1 dargestellt Nukleotidsequenz aufweist.

7. Rekombinanter Vektor, dadurch **gekennzeichnet,** daß er eine DNA-Sequenz nach einem der Ansprüche 1 bis 6 enthält.

8. Rekombinanter Vektor nach Anspruch 7, dadurch **gekennzeichnet,** daß er eine DNA-Sequenz nach einem der Ansprüche 1 bis 6 in der multi-purpose cloning site enthält.

9. Rekombinanter Vektor nach Anspruch 8, dadurch **gekennzeichnet,** daß er das Plasmid pUR 288C (DSM 4164) ist.

10. Rekombinanter Vektor nach Anspruch 8, dadurch **gekennzeichnet,** daß er das Plasmid pMS 103 (DSM 4165) ist.

11. Rekombinanter Vektor nach Anspruch 8, dadurch **gekennzeichnet,** daß er das Plasmid pUC 18C (DSM 4163) ist.

12. Wirtsorganismus, dadurch **gekennzeichnet,** daß er mit einem der rekombinanten Vektoren nach einem der Ansprüche 7 bis 11 transformiert ist.

13. Wirtsorganismus nach Anspruch 12, dadurch **gekennzeichnet,** daß er ein Bakterium, vorzugsweise ein Stamm der Art E.coli oder B.subtilis oder ein mikroskopisch kleiner Pilz, vorzugsweise der Art Aspergillus niger, Aspergillus oryzae oder Sacchaomyces cerevisiae ist.

14. Wirtsorganismus nach einem der Ansprüche 12 oder 13, dadurch **gekennzeichnenet,** daß er ausgewählt wird aus der Gruppe, die umfaßt: die E.coli-Stämme K12 JM 101 (ATCC 33876), K12 DH1 (ATCC 33849), K12 W6 (DSM 3632) und K12 JM 105 (DSM 4162) , der jeweils mit einem der rekombinanten Vektoren nach einem der Ansprüche 7 bis 11 tranformiert ist.

15. Protein mit den biologischen Eigenschaften des Cystatin C, dadurch **gekennzeichnet,** daß es weniger oder mehr als 120 Aminosäuren enthält.

16. Protein nach Anspruch 15, dadurch **gekennzeichnet,** daß es die in Abbildung 9 dargestellte Aminosäuresequenz hat.

17. Protein nach Anspruch 15, dadurch **gekennzeichnet,** daß es die Aminosäuresequenz

LEU VAL GLY GLY PRO MET ASP ALA SER VAL GLU GLU GLU GLY
VAL ARG ARG ALA LEU ASP PHE ALA VAL GLY GLU TYR ASN LYS
ALA SER ASN ASP MET TYR HIS SER ARG ALA LEU GLN VAL VAL
ARG ALA ARG LYS GLN ILE VAL ALA GLY VAL ASN TYR PHE LEU
ASP VAL GLU LEU GLY ARG THR THR CYS THR LYS THR GLN PRO
ASN LEU ASP ASN CYS PRO PHE HIS ASP GLN PRO HIS LEU LYS
ARG LYS ALA PHE CYS SER PHE GLN ILE TYR ALA VAL PRO TRP
GLN GLY THR MET THR LEU SER LYS SER THR CYS GLN ASP ALA

aufweist.

18. Protein nach Anspruch 15, dadurch **gekennzeichnet,** daß es ein Fusionsprotein aus Cystatin C und einem dem Wirtsorganismus nach Anspruch 12 eigenen Protein ist.

19. Protein nach Anspruch 18, dadurch **gekennzeichnet,** daß es das Cystatin C/β-Galactosidase-Fusionsprotein ist.

20. Verfahren zum Herstellen eines Proteins mit den biologischen Eigenschaften des Cystatin C, insbesondere eines Proteins nach einem der Ansprüche 15 bis 19, dadurch **gekennzeichnet,** daß man einen Wirtsorganismus nach einem der Ansprüche 12 bis 14 in einem üblichen Nährmedium züchtet, gegebenenfalls die Expression des Genprodukts induziert, das Expressionsprodukt aus der Kultur isoliert und gegebenenfalls in an sich bekannter Weise die nicht zur Aminosäuresequenz des Cystatin C gehörenden, terminal angeknüpften Aminosäuren, Peptide oder Polypeptide davon abspaltet.

21. Arzneimittel, **gekennzeichnet** durch einen Gehalt an einem Protein mit den biologischen Eigenschaften des Cystatin C und üblichen Träger- und/oder Hilfsstoffen und/oder Verdünnungsmitteln.

22. Verwendung eines Proteins nach einem der Ansprüche 15 bis 17 zum Herstellen von Arzneimitteln zur Therapie und/oder Prophylaxe von Virusinfektionen und Tumoren bei Mensch und Tier.

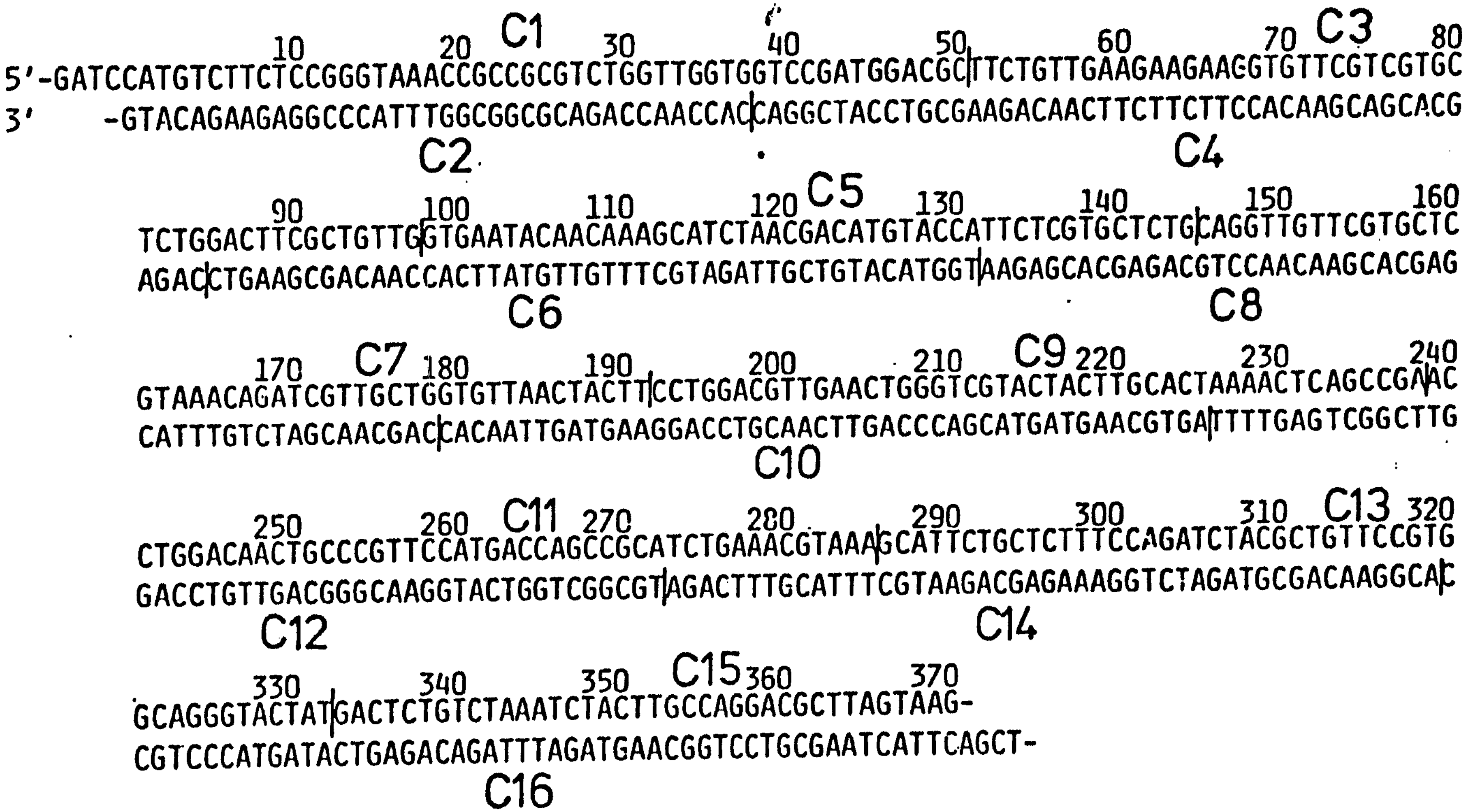


Fig. 1. DNA-Sequenz, die der Synthese und der Klonierung des Cystatin-C-Gens zugrunde gelegt wurde.

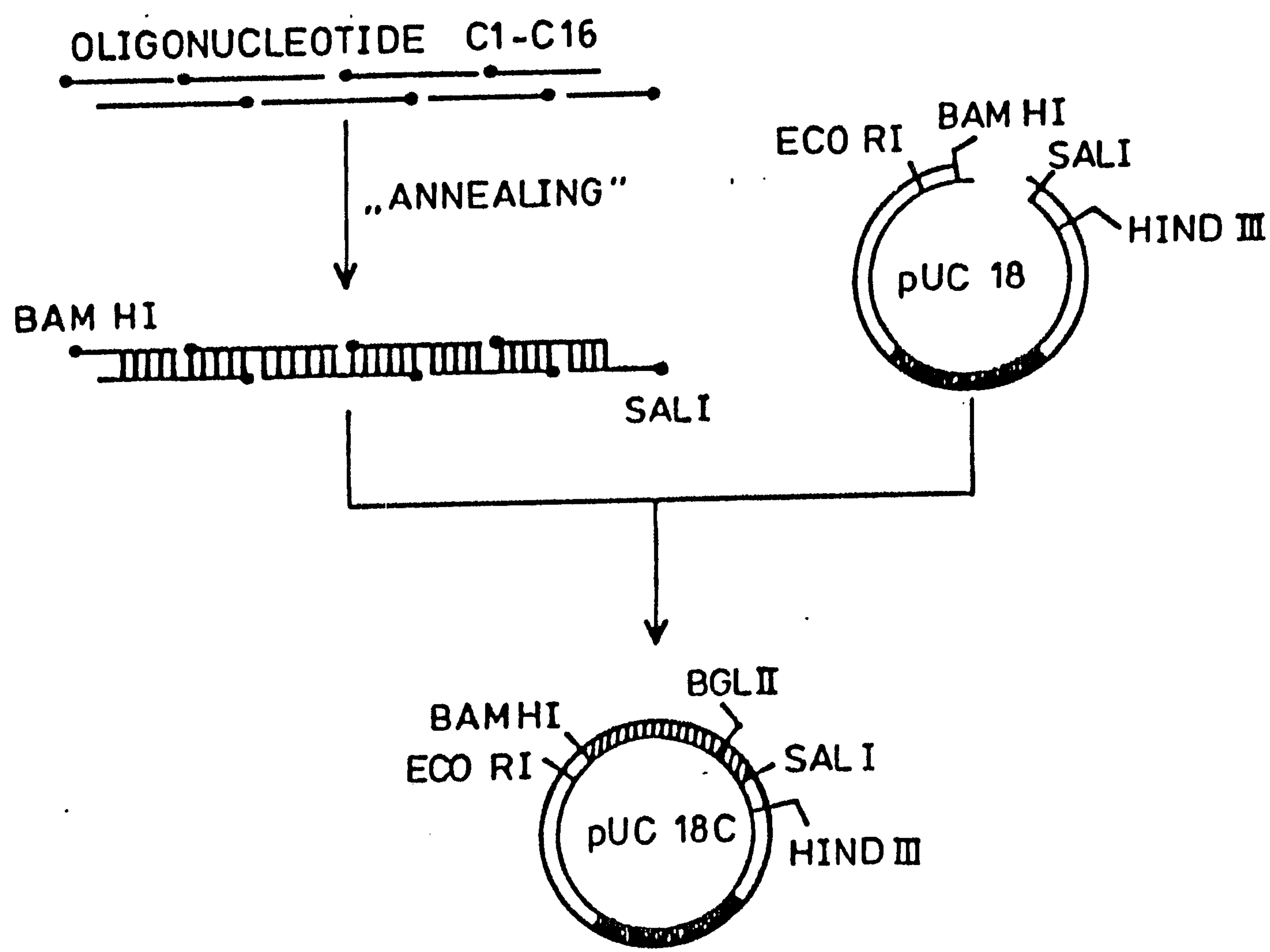


Fig. 2. Schema zur Synthesestrategie des Cystatin-C-Gens.

| | | |
|---|---|---|
| 1 | 5′ | GATCCATGTCTTCTCCGGGTAAACCGCCGCGTCTGGTTGGTGGTCCGATGGACGC |
| 2 | 3′ | GTACAGAAGAGGCCCATTTGGCGGCGCAGACCAACCAC |
| 3 | 5′ | TTCTGTTGAAGAAGAAGGTGTTCGTCGTGCTCTGGACTTCGCTGTTG |
| 4 | 3′ | CAGGCTACCTGCGAAGACAACTTCTTCTTCCACAAGCAGCACGAGAC |
| 5 | 5′ | GTGAATACAACAAAGCATCTAACGACATGTACCATTCTCGTGCTCTG |
| 6 | 3′ | CTGAAGCGACAACCACTTATGTTGTTTCGTAGATTGCTGTACATGGT |
| 7 | 5′ | CAGGTTGTTCGTGCTCGTAAACAGATCGTTGCTGGTGTTAACTACTT |
| 8 | 3′ | AAGAGCACGAGACGTCCAACAAGCACGAGCATTTGTCTAGCAACGAC |
| 9 | 5′ | CCTGGACGTTGAACTGGGTCGTACTACTTGCACTAAAACTCAGCCGA |
| 10 | 3′ | CACAATTGATGAAGGACCTGCAACTTGACCCAGCATGATGAACGTGA |
| 11 | 5′ | ACCTGGACAACTGCCCGTTCCATGACCAGCCGCATCTGAAACGTAAA |
| 12 | 3′ | TTTTGAGTCGGCTTGGACCTGTTGACGGGCAAGGTACTGGTCGGCGT |
| 13 | 5′ | GCATTCTGCTCTTTCCAGATCTACGCTGTTCCGTGGCAGGGTACTAT |
| 14 | 3′ | AGACTTTGCATTTCGTAAGACGAGAAAGGTCTAGATGCGACAAGGCA |
| 15 | 5′ | GACTCTGTCTAAATCTACTTGCCAGGACGCTTAGTAAG |
| 16 | 3′ | CCGTCCCATGATACTGAGACAGATTTAGATGAACGGTCCTGCGAATCATTCAGCT |

Fig. 3. Sequenzen der 16 Oligonucleotide, die bei der Synthese des Cystatin-C-Gens verwendet wurden.

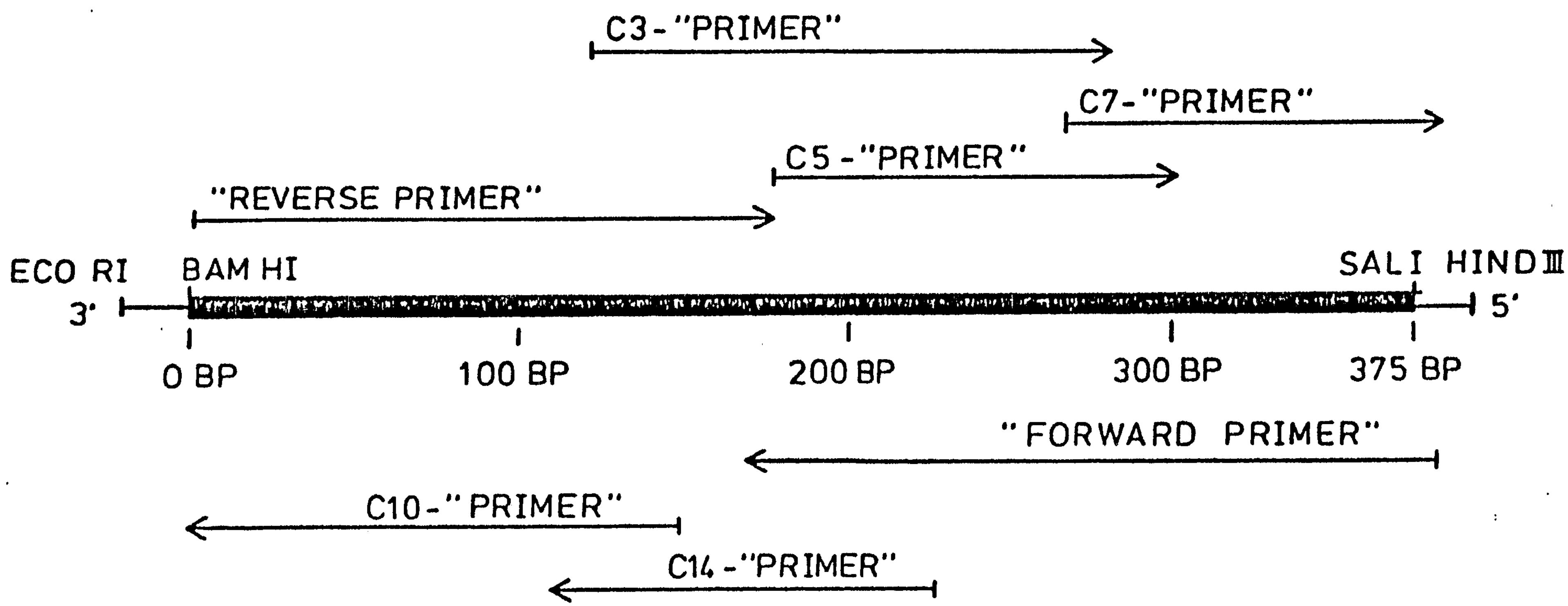


Fig. 4. Schema zur Sequenzierungsstrategie von pUC 18C.

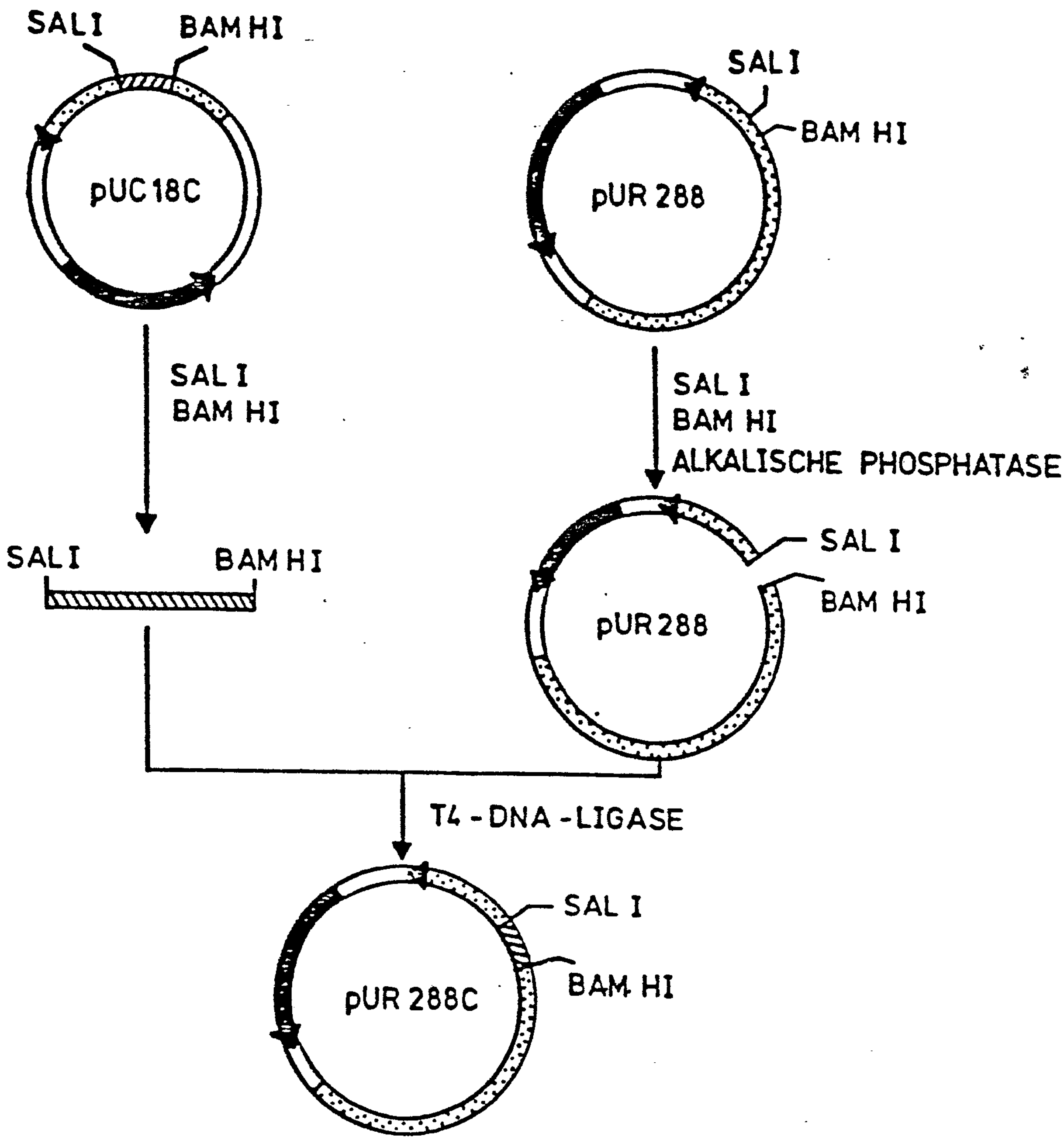


Fig. 5. Schema zur Konstruktion von pUR 288C.

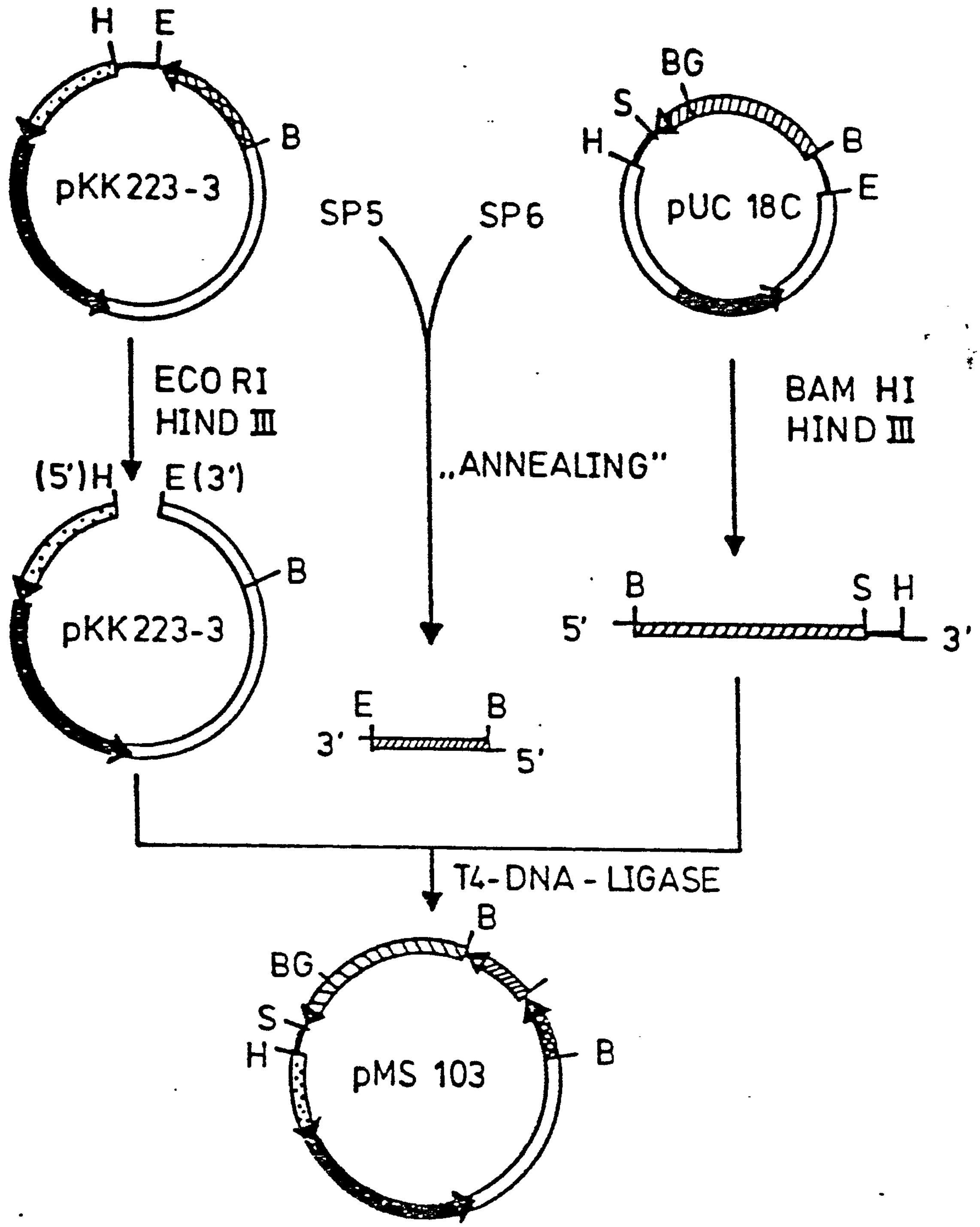


Fig. 6. Schema zur Konstruktion von pMS 103.

```
     S/D                 ECO RI*
5'   A G G A A A C A G A A T T GTG AAA CAA AGC ACT ATT GCA CTG GCA CTC TTA CCG TTA CTG TTT
     T C C T T T G T C T T A A CAC TTT GTT TCG TGA TAA CGT GAC CGT GAG AAT GGC AAT GAC AAA

                               VAL LYS GLN SER THR ILE ALA LEU ALA LEU LEU PRO LEU LEU PHE


                             BAM HI
     ACC CCT GTG ACA AAA GCA GGA TCC ATG TCT TCT CCG GGT AAA   3'
     TGG GGA CAC TGT TTT CGT CCT AGG TAC AGA AGA GGC CCA TTT

     THR PRO VAL THR LYS ALA GLY SER MET SER SER PRO GLY LYS
```

Fig. 7. Ausschnitt aus der DNA-Sequenz des Ligationsproduktes aus Beispiel 3a.

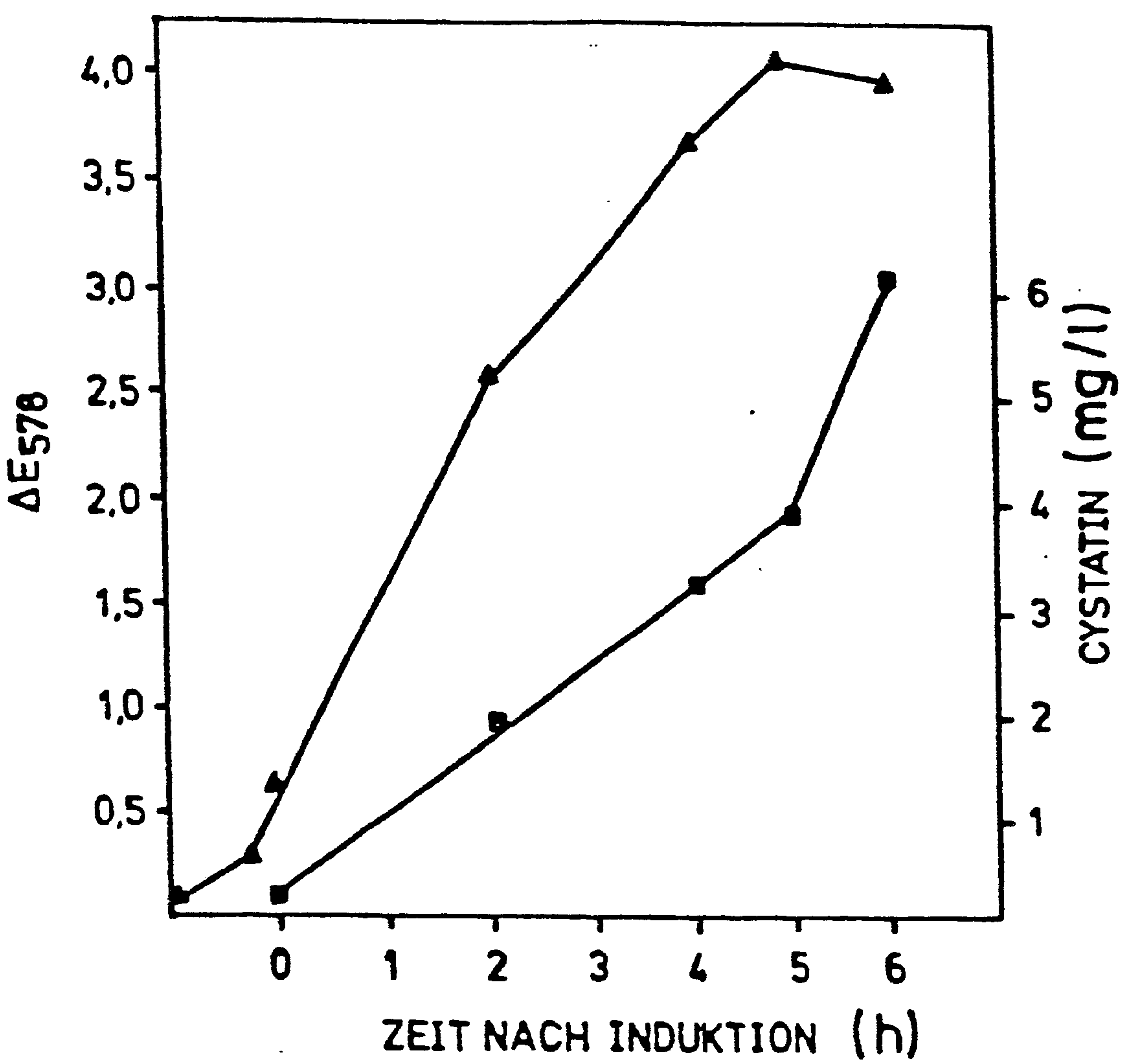


Fig. 8. Zeitlicher Verlauf von Zellwachstum und Cystatin-Expression nach Induktion mit ITPG in einer 8-l-Batchkultur.

```
   1                                         10                                  20
GLY SER MET SER SER PRO GLY LYS PRO PRO ARG LEU VAL GLY GLY PRO MET ASP ALA SER VAL GLU GLU
                                                 S
                                  30                                      40
GLU GLY VAL ARG ARG ALA LEU ASP PHE ALA VAL GLY GLU TYR ASN LYS ALA SER ASN ASP
                                     S
                                  50                                      60
MET TYR HIS SER ARG ALA LEU GLN VAL VAL ARG ALA ARG LYS GLN ILE VAL ALA GLY VAL
        S
                                     B1
                                  70                                      80
ASN TYR PHE LEU ASP VAL GLU LEU GLY ARG THR THR CYS THR LYS THR GLN PRO ASN LEU
                                     B1
                                  90                                      100
ASP ASN CYS PRO PHE HIS ASP GLN PRO HIS LEU LYS ARG LYS ALA PHE CYS SER PHE GLN
                                     B1
                                                                    T
                                  110                                     120
ILE TYR ALA VAL PRO TRP GLN GLY THR MET THR LEU SER LYS SER THR CYS GLN ASP ALA
                                                        B2
            T
```

Fig. 9. Vollständige Aminosäuresequenz von rekombinantem Human-Cystatin-C aus E.coli.

Europäisches Patentamt

# EUROPÄISCHER RECHERCHENBERICHT

Nummer der Anmeldung

EP 88 11 0975

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| O,X | THE FEBS SATELLITE MEETING ON PROTEINASE INHIBITORS AND BIOLOGICAL CONTROL, LJUBLJANA/BRDO, 4.-7. Juli 1987, Biol. Chem. Hoppe-Seyler, 1988, Band 369, Nr. "Suppl.", Seiten 205-208; J. GHISO et al.: "Isolation of a sequence encoding human cystatin C. Conservation of exon-intron structure between members of the cysteine proteinase inhibitors superfamily"<br>* Insgesamt * | 1,2,4 | C 12 N 15/00<br>C 12 N 1/20<br>C 12 P 21/02<br>C 07 K 13/00<br>A 61 K 37/02 //<br>(C 12 N 1/20<br>C 12 R 1:19 ) |
| Y | Idem<br>--- | 3,5,9-11 | |
| X | FEBS LETTERS, Band 216, Nr. 2, 1. Juni 1987, Seiten 229-233, Federation of European Biochemical Societies; M. ABRAHAMSON et al.: "Molecular cloning and sequence analysis of cDNA coding for the precursor of the human cysteine proteinase inhibitor cystatin C"<br>* Seite 230, Spalte 1, Zeilen 9-33; Seite 231, Figur 2; Seite 232, "Discussion" * | 1,2,4,6-8,12,13,14,20 | **RECHERCHIERTE SACHGEBIETE (Int. Cl.4)** |
| Y | Idem<br>--- | 3,5,9-11 | C 12 N<br>C 12 P<br>A 61 K |
| D,X | PROC. NATL. ACAD. SCI. USA, Band 79, Mai 1982, Seiten 3024-3027; A. GRUBB et al.: "Human gamma-trace, a basic microprotein: Amino acid sequence and presence in the adenohypophysis"<br>* Insgesamt *<br>-/- | 15,16,17 | |

Der vorliegende Recherchenbericht wurde für alle Patentansprüche erstellt

| Recherchenort | Abschlußdatum der Recherche | Prüfer |
|---|---|---|
| DEN HAAG | 21-10-1988 | PULAZZINI A.F.R. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 88 11 0975

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | Idem<br>--- | 3,5,9-11,18, 19,21, 22 | |
| D,X | BIOCHEM. BIOPHYS. RES. COMMUN., Band 120, Nr. 2, 30. April 1984, Seiten 631-636, Academic Press, Inc.; A.J. BARRETT et al.: "The place of human gamma-trace (cystatin C) amongst the cysteine proteinase inhibitors"<br>* Insgesamt * | 15,16, 17 | |
| D,Y | Idem<br>--- | 3,5,9-11,18, 19,21, 22 | |
| D,Y | EP-A-0 188 262 (KRKA)<br>* Seite 2, Spalte 2, Zeilen 1-22; Ansprüche 2,4,5,7 *<br>----- | 21,22 | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 21-10-1988 | PULAZZINI A.F.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)